(19) Europäisches Patentamt
European Patent Office
Office européen des brevets

(11) **EP 3 315 068 B1**

(12) **EUROPEAN PATENT SPECIFICATION**

(45) Date of publication and mention
of the grant of the patent:
**21.10.2020 Bulletin 2020/43**

(51) Int Cl.:
***A61B 5/11*** *(2006.01)*

(21) Application number: **16814062.2**

(22) Date of filing: **12.05.2016**

(86) International application number:
**PCT/JP2016/064139**

(87) International publication number:
**WO 2016/208291 (29.12.2016 Gazette 2016/52)**

(54) **DEVICE, METHOD, AND COMPUTER PROGRAM FOR PROVIDING POSTURE FEEDBACK DURING AN EXERCISE**

VORRICHTUNG, VERFAHREN UND COMPUTERPROGRAMM ZUR BEREITSTELLUNG VON HALTUNGSFEEDBACK WÄHREND EINER ÜBUNG

DISPOSITIF, PROCÉDÉ ET PROGRAMME D'ORDINATEUR POUR FOURNIR UNE RÉTROACTION DE POSTURE PENDANT UN EXERCICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2015 JP 2015129019**

(43) Date of publication of application:
**02.05.2018 Bulletin 2018/18**

(73) Proprietor: **NEC Solution Innovators, Ltd.**
**Tokyo 136-8627 (JP)**

(72) Inventors:
• **NODA Hisashi**
**Tokyo 136-8627 (JP)**
• **NAGAI Katsuyuki**
**Tokyo 136-8627 (JP)**
• **KINOSHITA Tomomi**
**Tokyo 136-8627 (JP)**
• **TERASHIMA Hiroki**
**Tokyo 136-8627 (JP)**

(74) Representative: **Betten & Resch**
**Patent- und Rechtsanwälte PartGmbB**
**Maximiliansplatz 14**
**80333 München (DE)**

(56) References cited:
**JP-A- 2002 000 584      JP-A- 2014 138 661**
**JP-A- 2015 042 241      US-A1- 2008 161 733**
**US-A1- 2015 003 687**

• **RICHARD TANG ET AL: "Physio@Home", HUMAN FACTORS IN COMPUTING SYSTEMS, ACM, 2 PENN PLAZA, SUITE 701 NEW YORK NY 10121-0701 USA, 18 April 2015 (2015-04-18), pages 4123-4132, XP058068292, DOI: 10.1145/2702123.2702401 ISBN: 978-1-4503-3145-6**
• **Dalibor Kiseljak ET AL: "ASSESSMENT OF THE QUALITY OF MOVEMENT FOR PATIENTS WITH ADOLESCENT IDIOPATHIC SCOLIOSIS", ACTA UNIVERSITATIS CAROLINAE KINANTHROPOLOGICA, 1 January 2014 (2014-01-01), pages 56-68, XP055528288, Retrieved from the Internet: URL:https://bib.irb.hr/datoteka/698938.KIN ANTHROPOLOGICA_Vol._50_2__2014.pdf [retrieved on 2018-11-29]**
• **ALANA DA GAMA ET AL: "Guidance and Movement Correction Based on Therapeutics Movements for Motor Rehabilitation Support Systems", VIRTUAL AND AUGMENTED REALITY (SVR), 2012 14TH SYMPOSIUM ON, IEEE, 28 May 2012 (2012-05-28), pages 191-200, XP032234906, DOI: 10.1109/SVR.2012.15 ISBN: 978-1-4673-1929-4**
• **YOHEI OKADA: "In-Home Posture Evaluation and Visual Feedback Training to Improve Posture with a Kinect-Based System in Parkinson's Disease", JOURNAL OF NOVEL PHYSIOTHERAPIES, vol. 04, no. 05, 1 January 2014 (2014-01-01), XP055527881, DOI: 10.4172/2165-7025.1000232**

- KYOZO YONEMOTO ET AL.: 'KANSETSU KADOIKI HYOJI NARABINI SOKUTEIHO (HEISEI 7 NEN 4 GATSU KAITEI)' JAPANESE JOURNAL OF REHABILITATION MEDICINE vol. 32, no. 4, 01 April 1995, pages 207 - 217, XP055505070

**Description**

TECHNICAL FIELD

The present invention relates to a technique for measuring an ability of a limb of a person.

BACKGROUND ART

At present, there are various applications for three-dimensionally capturing a motion of a person by using a motion capture technique or a three-dimensional sensing technique and converting the motion into digital data. An application for generating a Computer Graphics (CG) character in a movie or a game and performing sports analysis, an application for recognizing a gesture operation, and the like are exemplified. A motion capture method includes a method in which a plurality of markers or sensors are attached to a human body, and the human's motion is converted into three-dimensional data by the detection of the markers or the sensing of the sensors. In addition, the motion capture method also includes a method in which the posture of the human body is estimated using three-dimensional information obtained from a three-dimensional sensor so that the human's motion is converted into three-dimensional data without attaching a marker or a sensor to the human body. In the estimation of the posture of the human body, portions (head, hand, arm, leg, and the like) of the human's limbs are recognized from the three-dimensional information, and motions of the portions are recorded.

Patent Document 1 described below proposes a device that measures an index value which is an index for evaluating rehabilitation whenever operation information (color image information, distance image information, framework information, and the like) of a person is acquired on the basis of information obtained from Kinect (registered trademark). In this proposal, an index of at least one of flexion extension of a shoulder, horizontal flexion extension of the shoulder, flexion extension of a shoulder girdle, flexion extension of a hip, forward flexion and backward flexion of a head, forward flexion and backward flexion of a thoracic and lumbar spine, and a rotation motion of a thoracic and lumbar spine is measured.

In Non-Patent Document 1 described below, a correct method for measuring a joint movable range is determined.

Non-Patent Document 2 described below proposes a method of measuring a joint movable range in real time by using framework information obtained from KINECT (registered trademark). In this method, the framework information is displayed, and a measurement item in the joint is displayed when a mouse is superimposed on the joint to be measured. The measurement is started by selecting a desired measurement item. However, this document does not disclose a concrete method of the measurement.

RELATED DOCUMENT

PATENT DOCUMENT

[Patent Document 1] Japanese Patent Application Publication No. 2015-61579

NON-PATENT DOCUMENT

[Non-Patent Document 1] "Joint Movable Range Display and Measurement Method", Japanese Orthopaedic Assoc. and Japanese Assoc. of Rehabilitation Medicine, Rehabilitation Medicine Vol.32, pp.207-217, 1995
[Non-Patent Document 2] Kitsunezaki Naofumi, etc., "KINECT Applications for the Physical Rehabilitation", the Institute of Electronics, Information and Communication Engineers, IEICE technical report, IE2012-89 (2012-11)

The document: "RICHARD TANG ET AL, "Physio@Home", HUMAN FACTORS IN COMPUTING SYSTEMS, ACM, 2 PENN PLAZA, SUITE 701 NEW YORK NY 10121-0701 USA, (20150418), doi:10.1145/2702123.2702401, ISBN 978-1-4503-3145-6" relates to exploring visual guidance and feedback techniques for physiotherapy exercises.

SUMMARY OF THE INVENTION

TECHNICAL PROBLEM

However, in the above-proposed methods, an index value for evaluating rehabilitation may not be accurately measured. In order to correctly measure the index value, it is necessary for a test subject to perform a determined motion in a correct posture. For example, in a case where measurement is performed in a state where a spine is bent forward and backward during a flexion motion of a shoulder, a correct evaluation result is not obtained.

The above-described problem will be described in more detail on the basis of a more specific scene. For example, a scene is assumed in which the index value for the evaluation is automatically measured by the test object using a system using the above-described proposed method. The test subject does not know a correct posture or motion to be measured at a point in time when the test subject is not accustomed to using the system, and thus there is a strong possibility that a correct value cannot be measured even when the system is used. Even when the test subject remembers a correct posture or motion to a certain degree, the test subject does not always take a correct posture during measurement. Human beings tend to unconsciously take a posture or motion which is easy for themselves due to a habit of the body, or the like, and thus there is a possibility that the test subject unconsciously takes an erroneous posture or motion even when the test subject intends to take a correct posture or motion. In addition, also in a scene where the system is used by a measuring person attended, a correct value is not necessarily measured. There are a plurality of examination items for determining a correct posture, and the measuring person has a great burden to sequentially check the plurality of examination items while the posture of the test subject is constantly changing. Accordingly, a human error such as overlook or erroneous determination is likely to occur in checking the posture or motion by the measuring person, and the measuring person's subjectivity is also included, which leads to a possibility that variations occur in evaluation.

The present invention is contrived in view of such situations, and an object thereof is to provide a technique for accurately measuring the ability of a limb a test subject.

SOLUTION TO PROBLEM

The invention is defined in the independent claims.

In examples, the following configurations are adopted in order to solve the above-described problems.

A first example relates to a measurement device. The measurement device according to the first aspect includes an acquisition unit that acquires positional information or direction information on a predetermined portion in a limb of a test subject, a calculation unit that calculates angles or distances serving as ability indexes of the limb of the test subject on the basis of the acquired positional information or direction information, a determination unit that determines whether or not a posture of the test subject is proper on the basis of the acquired positional information or direction information, and an output processing unit that outputs the calculated angles or distances in a state corresponding to a determination result regarding whether or not the posture of the test subject is proper.

A second example relates to a measurement method executed by at least one computer. The measurement method according to the second aspect includes acquiring positional information or direction information on a predetermined portion in a limb of a test subject, calculating angles or distances serving as ability indexes of the limb of the test subject on the basis of the acquired positional information or direction information, determining whether or not a posture of the test subject is proper on the basis of the acquired positional information or direction information, and outputting the calculated angles or distances in a state corresponding to a determination result regarding whether or not the posture of the test subject is proper.

Meanwhile, another example relates to a program for causing at least one computer to execute the method of the second aspect, or may relate to a storage medium readable by the computer having the program recorded thereon. This storage medium includes a non-transitory tangible medium.

ADVANTAGEOUS EFFECTS OF INVENTION

According to the above-described aspects, it is possible to provide a technique for accurately measuring the ability of a limb of a test subject.

BRIEF DESCRIPTION OF THE DRAWINGS

The above-described objects, other objects, features and advantages will be further apparent from the preferred example embodiments described below, and the accompanying drawings as follows.
FIG. 1 is a schematic diagram illustrating an example of a hardware configuration of a measurement device according to a first example embodiment.
FIG. 2 is a schematic diagram illustrating an example of a processing configuration of the measurement device according to the first example embodiment.
FIG. 3 is a diagram illustrating a method of calculating an ability index of a shoulder.
FIG. 4 is a diagram illustrating an example of the output of a display when the ability of a flexion motion of the shoulder is measured.

FIG. 5 is a diagram illustrating an example of the output of a display when the ability of an abduction motion of the shoulder is measured.

FIG. 6 is a diagram illustrating an example of the output of a display when the ability of an external rotation motion of the shoulder is measured.

FIG. 7 is a diagram illustrating an example of the output of a display when the ability of a horizontal flexion motion of the shoulder is measured.

FIG. 8 is a flow chart illustrating an example of the operation of the measurement device according to the first example embodiment.

FIG. 9 is a diagram illustrating a method of calculating an ability index related to a flexion motion of a thoracic and lumbar spine.

FIG. 10 is a diagram illustrating an example of the output of a display when the ability of the flexion motion of the thoracic and lumbar spine is measured.

FIG. 11 is a diagram illustrating an example of the output of a display when the ability of the flexion motion of the thoracic and lumbar spine is measured.

FIG. 12 is a flow chart illustrating an example of the operation of a measurement device according to a second example embodiment.

FIG. 13 is a flow chart illustrating an example of the operation of the measurement device according to the second example embodiment.

FIG. 14 is a diagram illustrating an example of the output of a display when the ability of a functional reach test is measured.

FIG. 15 is a flow chart illustrating an example of the operation of a measurement device according to a third example embodiment.

FIG. 16 is a flow chart illustrating an example of the operation of the measurement device according to the third example embodiment.

FIG. 17 is a schematic diagram illustrating an example of a processing configuration of a measurement device according to a fourth example embodiment.

FIG. 18 is a diagram illustrating a method of calculating an ability index of a neck.

FIG. 19 is a diagram illustrating an example of the output of a display when a neck movable range is measured.

FIG. 20 is a flow chart illustrating an example of the operation of the measurement device according to the fourth example embodiment.

FIG. 21 is a diagram illustrating a method of calculating an ability index of a hip.

FIG. 22 is a diagram illustrating an example of the output of a display by an output processing unit according to a supplementary example.

FIG. 23 is a schematic diagram illustrating an example of a processing configuration of a measurement device according to a fifth example embodiment.

FIG. 24 is a flow chart illustrating an example of the operation of the measurement device according to the fifth example embodiment.

DESCRIPTION OF EXAMPLE EMBODIMENTS

Hereinafter, example embodiments of the invention will be described. Meanwhile, the example embodiments described below are merely illustrative of the invention, and the invention is not limited to the configurations of the following example embodiments.

[First Example embodiment]

[Configuration of Apparatus]

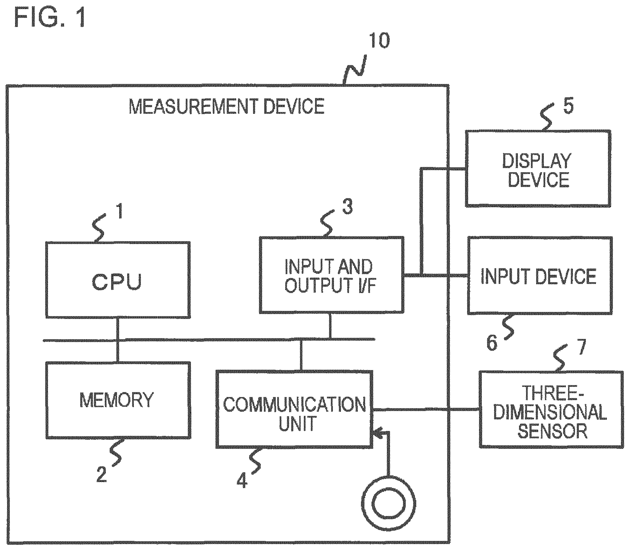

FIG. 1 is a schematic diagram illustrating an example of a hardware configuration of a measurement device 10 according to a first example embodiment. The measurement device 10 according to the first example embodiment is a so-called computer, and includes a Central Processing Unit (CPU) 1, a memory 2, an input and output interface (I/F) 3, a communication unit 4, and the like which are connected to each other, for example, via a bus.

The CPU 1 includes an application-specific integrated circuit (ASIC), a Digital Signal Processor (DSP), a Graphics Processing Unit (GPU), and the like in addition to a general CPU.

The memory 2 is a Random Access Memory (RAM), a Read Only Memory (ROM), or an auxiliary storage device (hard disk or the like).

The input and output I/F 3 can be connected to user interface devices such as a display device 5 and an input device 6. The display device 5 is a device, such as a Liquid Crystal Display (LCD) or a Cathode Ray Tube (CRT) display, which displays a screen corresponding to drawing data processed by the CPU 1 and the like. The input device 6 is a device which receives an input of a user operation, such as a keyboard or a mouse. The display device 5 and the input device 6 are integrated with each other, and may be realized as a touch panel. In addition, the input device 6 may be a microphone unit that acquires a voice. Another output device such as a speaker unit may be connected to the input and output I/F 3.

The communication unit 4 performs communication with another computer through a communication net (not shown), the transmission and reception of a signal to and from another devices such as a printer, and the like. The communication unit 4 is connected to a three-dimensional sensor 7 through a Universal Serial Bus (USB), or the like. However, a mode of communication between the communication unit 4 and the three-dimensional sensor 7 is not limited. In addition, a portable storage medium and the like may be connected to the communication unit 4.

The three-dimensional sensor 7 detects three-dimensional information. The three-dimensional sensor 7 is realized as a sensor in which a visible light camera and a depth sensor are integrated with each other, such as Kinect (registered trademark) or a 3D camera. The depth sensor is also referred to as a distance image sensor, and a distance between the distance image sensor and an object is calculated on the basis of information obtained by irradiating the object with a pattern of near-infrared light from a laser and capturing an image of the pattern by a camera detecting near-infrared light. The realization method of the three-dimensional sensor 7 is not limited as long as the three-dimensional sensor can detect a three-dimensional position of a predetermined portion of a test subject within a visual field. For example, the three-dimensional sensor 7 may be realized by a three-dimensional scanner system using a plurality of visible light cameras. In the following description, for convenience of description, it is assumed that the three-dimensional sensor 7 is a sensor in which a visible light camera and a depth sensor are integrated with each other.

A hardware configuration of the measurement device 10 is not limited to the example illustrated in FIG. 1. The measurement device 10 may include other hardware components not shown in the drawing. In addition, the number of hardware components is not also limited to the example in FIG. 1. For example, the measurement device 10 may include a plurality of CPUs 1.

[Processing Configuration]

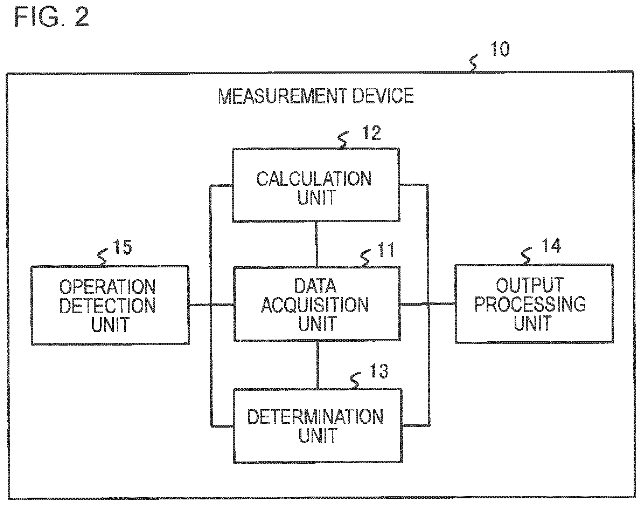

FIG. 2 is a schematic diagram illustrating an example of a processing configuration of the measurement device 10 according to the first example embodiment. The measurement device 10 according to the first example embodiment includes a data acquisition unit 11, a calculation unit 12, a determination unit 13, an output processing unit 14, an operation detection unit 15, and the like. These processing modules are realized, for example, by the CPU 1 executing programs stored in the memory 2. In addition, the programs may be installed through the communication unit 4 from a portable storage medium, such as a Compact Disc (CD) or a memory card, or another computer on a network, and may be stored in the memory 2.

The operation detection unit 15 detects a user operation. Specifically, the operation detection unit 15 detects a user operation with respect to the input device 6, on the basis of information input from the input and output I/F 3. For example, the operation detection unit 15 detects an operation of selecting a single motion type among a plurality of motion types which are determined in advance for the measurement of a shoulder movable range. In this example embodiment, the operation detection unit 15 detects a user operation for selecting one motion type out of a flexion motion, an extension motion, an abduction motion, an adduction motion, an external rotation motion, an internal rotation motion, a horizontal extension motion, and a horizontal flexion motion of a shoulder. The plurality of motion types may include a motion type requiring measurement with respect to each of the right and the left. Note that, the motions for measurement of a motion type of the shoulder being a target in this example embodiment require measurement for each of the right and the left. In a case where a motion type requiring measurement for the right and the left is selected, the operation detection unit 15 may detect an operation of selecting whether the right or the left is measured, together with the motion type. The operation detection unit 15 transmits the motion type selected by the user to the data acquisition unit 11, the calculation unit 12, and the determination unit 13 on the basis of the detected user operation.

The data acquisition unit 11 acquires positional information on a plurality of predetermined portions of limbs of the test subject on the basis of the information obtained from the three-dimensional sensor 7. The data acquisition unit 11 may acquire only positional information on a predetermined portion which is used by the calculation unit 12 and the determination unit 13, or may acquire pieces of positional information on predetermined portions as many as possible. The positional information to be acquired is represented by a world coordinate system equivalent to a three-dimensional space where the test subject is actually present. For example, the positional information to be acquired is represented by a world coordinate system in which a horizontal direction is set to be an x-axis (right direction is positive), a vertical direction is set to be a y-axis (upper direction is positive), and a depth direction (interior direction is positive) is set to be a z-axis, with the center of a visual field of the three-dimensional sensor 7 as the origin. Hereinafter, a description will be given using an example of the world coordinate system. However, the arrangement of the three axes and starting point of the world coordinate system is not limited to such an example.

For example, the data acquisition unit 11 acquires a frame of a two-dimensional image and a frame of a depth image (distance image) from the three-dimensional sensor 7 at a predetermined cycle. Both the frames may be acquired at the same cycle, or may be acquired at different cycles. Hereinafter, the frames are simply referred as a two-dimensional image and a depth image, respectively. The data acquisition unit 11 recognizes the plurality of predetermined portions of the limbs of the test subject which are positioned within the visual field of the three-dimensional sensor 7 from the acquired two-dimensional image and depth image by using a posture estimation technique, and determines a three-dimensional position (world coordinate system) of each of the portions. The data acquisition unit 11 can use various existing posture estimation techniques. For example, the data acquisition unit 11 can acquire pieces of positional information on a plurality of portions of limbs such as a left shoulder, a left elbow, a left wrist, a left hand, a right shoulder, a right elbow, a right wrist, a right hand, a head, the middle of the shoulder, a spine, the middle of a waist, a right waist, a left waist, a right knee, a right heel, a right foot, a left knee, a left heel, and a left foot. Hereinafter, the pieces of positional information on the predetermined portions of the limbs of the test subject which are acquired by the data acquisition unit 11 may be referred to as framework data. The data acquisition unit 11 sequentially acquires the framework data whenever a frame is acquired.

The calculation unit 12 sequentially calculates angles serving as ability indexes of the shoulder of the test subject on the basis of the pieces of framework data sequentially acquired by the data acquisition unit 11. The calculation unit 12 calculates an angle serving as an ability index of a motion type selected by the user among a plurality of motion types which are determined in advance for the measurement of a shoulder movable range. For example, the calculation unit 12 calculates an angle serving as an ability index of a flexion motion, an extension motion, an abduction motion, an adduction motion, an external rotation motion, an internal rotation motion, a horizontal extension motion, or a horizontal flexion motion of the shoulder. The angle to be calculated indicates a shoulder movable range. In a case where a motion type requiring the measurement of the right and the left is selected, the calculation unit 12 may automatically determine a direction (left or right) to be measured, or may determine the direction by a user operation detected by the operation detection unit 15.

The calculation unit 12 calculates an angle (shoulder movable range) by using the following method based on regulations described in Non-Patent Document 1.

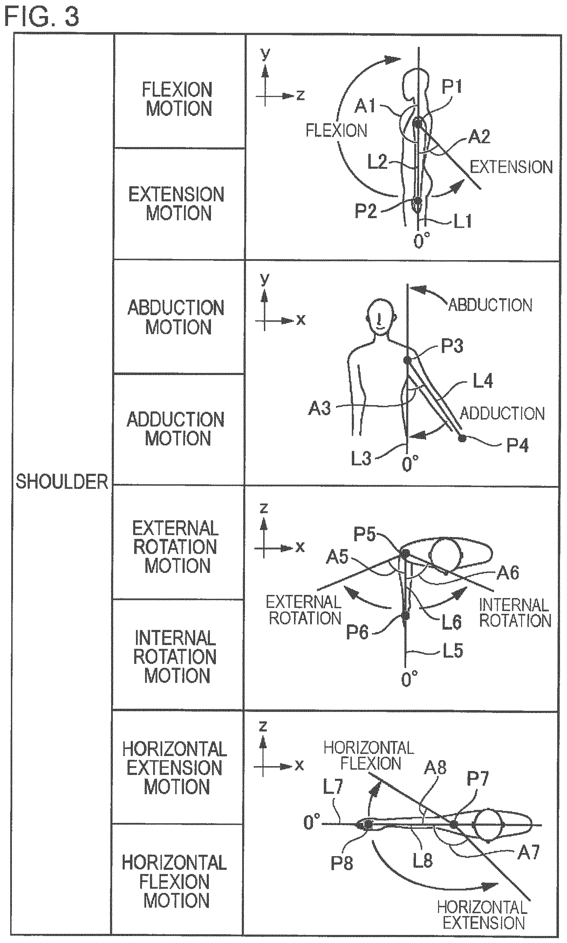

The calculation unit 12 calculates an angle on a plane perpendicular to the x-axis or z-axis as an ability index of a flexion motion, an extension motion, an abduction motion, or an adduction motion of the shoulder on the basis of the acquired framework data, the angle being formed by a line segment in the negative direction of the y-axis with the position of the shoulder as an endpoint and a line segment connecting the position of the shoulder and the position of the hand. In addition, the calculation unit 12 calculates an angle on a plane perpendicular to the y-axis as an ability index of an external rotation motion or an internal rotation motion of the shoulder on the basis of the acquired framework data, the angle being formed by a line segment in the front direction of the z-axis with the position of the elbow as an endpoint and a line segment connecting the position of the elbow and the position of the hand. In addition, the calculation unit 12 calculates an angle on a plane perpendicular to the y-axis as an ability index of a horizontal extension motion or a horizontal flexion motion of the shoulder on the basis of the acquired framework data, the angle being formed by a line segment in the x-axis direction with the position of the shoulder as an endpoint and a line segment connecting the position of the shoulder and the position of the hand. This calculation processing will be described in more detail with reference to FIG. 3.

FIG. 3 is a diagram illustrating a method of calculating an ability index of the shoulder. However, FIG. 3 illustrates only a method of calculating an ability index of a single shoulder out of the right shoulder and the left shoulder. An ability index of the shoulder on the other side not illustrated in FIG. 3 can be calculated on the basis of the same idea.

As an ability index of a flexion motion of the shoulder, the calculation unit 12 calculates an angle A1 on a plane (yz plane, the paper of FIG. 3) which is perpendicular to the x-axis, the angle being formed by a line segment L1 in the negative direction of the y-axis with the position P1 of the shoulder as an endpoint and a line segment L2 connecting a position P1 of the shoulder and a position P2 of the hand.

As an ability index of an extension motion of the shoulder, the calculation unit 12 calculates an angle A2 on a plane (yz plane, the paper of FIG. 3) which is perpendicular to the x-axis, the angle being formed by the line segment L1 and the line segment L2.

As an ability index of an abduction motion or an adduction motion of the shoulder, the calculation unit 12 calculates an angle A3 on a plane (xy plane, the paper of FIG. 3) which is perpendicular to the z-axis, the angle being formed by a line segment L3 in the negative direction of the y-axis with the position P3 of the shoulder as an endpoint and a line segment L4 connecting a position P3 of the shoulder and a position P4 of the hand.

As an ability index of an external rotation motion of the shoulder, the calculation unit 12 calculates an angle A5 on a plane (xz plane, the paper of FIG. 3) which is perpendicular to the y-axis, the angle being formed by a line segment L5 in the negative direction of the z-axis and a line segment L6 connecting a position P5 of the elbow and a position P6 of the hand with the position P5 of the elbow with the position P5 of the elbow as an endpoint.

As an ability index of an internal rotation motion of the shoulder, the calculation unit 12 calculates an angle A6 on a plane (xz plane, the paper of FIG. 3) which is perpendicular to the y-axis, the angle being formed by the line segment L5 and the line segment L6.

As an ability index of a horizontal flexion motion of the shoulder, the calculation unit 12 calculates an angle A7 on a plane (xz plane, the paper of FIG. 3) which is perpendicular to the y-axis, the angle being formed by a line segment L7 in the negative direction of the x-axis with the position P7 of the shoulder as an endpoint and a line segment L8 connecting a position P7 of the shoulder and a position P8 of the hand.

As an ability index of a horizontal extension motion of the shoulder, the calculation unit 12 calculates an angle A8 on a plane (xz plane, the paper of FIG. 3) which is perpendicular to the y-axis, the angle being formed by the line segment L7 and the line segment L8.

The determination unit 13 determines the propriety of a posture of the test subject on the basis of the framework data acquired by the data acquisition unit 11. For example, the determination unit 13 detects the body being bent back and forth, the elbow being bent, the shoulder not being horizontal, any one of shoulders being moved back and forth, and the like in a case where an ability index value of a flexion motion, an extension motion, an abduction motion, an adduction motion, a horizontal extension motion, or a horizontal flexion motion of the shoulder is calculated by the calculation unit 12, and thus the determination unit determines that the posture of the test subject is improper. In a case where these motions are not detected, the determination unit 13 determines that the posture of the test subject is proper. In addition, the determination unit 13 detects the body being bent back and forth, the elbow not being bent at 90 degrees, the shoulder of a moving arm being pulled to the back side, and the like in a case where an ability index value of an external rotation motion or an internal rotation motion in the shoulder movable range is calculated by the calculation unit 12, and thus the determination unit determines that the posture of the test subject is improper. However, the posture of the test subject which is determined by the determination unit 13 is not limited to such examples.

Specifically, on the basis of framework data of the head, the middle of the shoulder, the spine, and the middle of the waist, the determination unit 13 calculates deviation degrees of positions on the z-axis of the head, the middle of the shoulder, the spine, and the middle of the waist. In a case where the calculated deviation degree exceeds a predetermined distance, the determination unit 13 determines that the posture of the test subject is improper since the body is bent back and forth. In addition, the determination unit 13 calculates an angle of the elbow on the basis of the framework data of the shoulder, the elbow, and the hand, and determines that the posture of the test subject is improper in a case where the angle of the elbow is equal to or less than a predetermined angle since the test subject bends the elbow. In addition, the determination unit 13 calculates an angle formed by a line segment connecting both shoulders and the x-axis on the xz plane on the basis of framework data of both shoulders, and determines that the posture of the test subject is improper on the assumption that the shoulder of a moving arm is pulled to the back side in a case where the angle exceeds a predetermined angle. The determination unit 13 calculates the angle of the elbow on the basis of framework data of the shoulder, the elbow, and the hand, and determines that the posture of the test subject is improper in a case where the angle of the elbow is less than 90 degrees since the elbow is not bent at 90 degrees.

The output processing unit 14 outputs an angle calculated by the calculation unit 12 in a state corresponding to a determination result regarding whether or not the posture of the test subject is proper. For example, the output processing unit 14 outputs the angle calculated by the calculation unit 12 with a color corresponding to the determination result of the determination unit 13. In a case where the determination unit 13 determines that the posture of the test subject is improper, the output processing unit 14 outputs an angle with a black color, the angle being calculated from framework data acquired at the same time as framework data used for the determination. In a case where it is determined that the posture is improper, the output processing unit 14 outputs an angle at the time with a red color. As another example, in a case where it is determined that the posture is improper, the output processing unit 14 may output an angle by flickering the angle, may change a background color of the angle, or may output a predetermined voice together with the output of a display of the angle.

In addition, the output processing unit 14 can also output a display including an image with the test subject, on which line segment connecting a plurality of predetermined portions corresponding to framework data used for the calculation of the angle is super imposed in a state corresponding to a determination result regarding whether or not the posture of the test subject is proper. For example, the output processing unit 14 outputs a display including the image with the test subject, on which the line segment colored with a color corresponding to the determination result of the determination unit 13 is superimposed. As another example, the output processing unit 14 may output the line segment by flickering the line segment or may display the line segment by making the line segment thin in a case where it is determined that the posture is improper.

The output processing unit 14 converts positional information on a world coordinate system indicated by framework data into positional information on an image coordinate system of a two-dimensional image obtained from the three-dimensional sensor 7, and thus it is possible to superimpose a mark (image element) on a plurality of predetermined portions of the test subject included in the two-dimensional image. For example, it is preferable that the output processing unit 14 attaches a mark to at least one of the predetermined portions used for the calculation of and the predetermined portions used for the determination of the propriety of the posture. Thereby, it is possible to make the test subject easily recognize which portion relates to measurement with respect to a target motion type. Further, the output processing unit 14 can further superimpose a line segment connecting the plurality of predetermined portions on the image on the basis of a correspondence relation between the world coordinate system indicated by the framework data and the image coordinate system of the two-dimensional image. Since the test subject easily recognize his or her own posture being measured, such as the arm being bent or both shoulders being inclined, on the basis of the line segment, it is possible to lead the test subject to a correct posture.

Further, the output processing unit 14 may output a maximum value among angles that are sequentially calculated by the calculation unit 12. For example, the output processing unit 14 can also exclude an angle calculated when it is determined that the posture of the test subject is improper from selection candidates of the maximum value while outputting the maximum value. In addition, the output processing unit 14 can also hold a finally determined maximum value as a measured value for each measurement, and can also output the smallest value, among measured values held by that time, as a minimum value. The output processing unit 14 outputs 0 degrees as a minimum value during first measurement. In addition, in a case where an output maximum value is smaller than a minimum value during each measurement after second measurement, the output processing unit 14 may output the maximum value as a minimum value. Here, a section between the start of measurement and the termination of measurement is counted as a single measurement, and the start of measurement and the termination of measurement are determined, for example, by a user operation detected by the operation detection unit 15.

Incidentally, an output method of the output processing unit 14 is not limited. The output processing unit 14 may display the above-described information on the display device 5, or may cause a printing device connected to the communication unit 4 to print the information.

[Example of Output]

Next, a specific example of the output of a display by the output processing unit 14 according to the first example embodiment will be described.

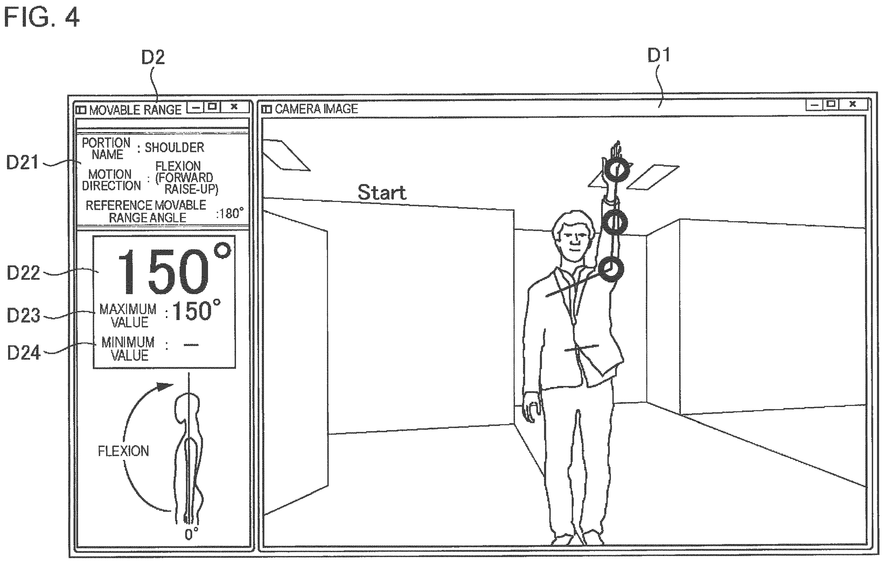

FIG. 4 is a diagram illustrating an example of the output of a display when an ability of a flexion motion of the shoulder is measured. According to the example in FIG. 4, the output processing unit 14 output a video area D1 and an explanation area D2 so as to be adjacent to each other. The output processing unit 14 sequentially displays two-dimensional images obtained from the three-dimensional sensor 7 in the video area D1. At this time, the output processing unit 14 superimposes a mark (doughnut-shaped image) on the plurality of predetermined portions corresponding to framework data used for the calculation of the angle on the two-dimensional image displayed in the video area D1. Further, the output processing unit 14 superimposes a line segment connecting the plurality of predetermined portions on the two-dimensional image. In the example in FIG. 4, a doughnut-shaped mark is displayed on the left hand, the left elbow, and the left shoulder, and a line segment connecting the left hand, the left elbow, the left shoulder, and the right shoulder and a line segment connecting the left waist and the right waist is displayed. The left elbow, the right shoulder, the left waist, and the right waist are not directly used for the calculation of the angle, but a mark or a line segment is shown in order to promote a correct posture to the test subject. For example, both shoulders being horizontal and the heights of the right and left waists being uniform are desired as a correct posture.

The explanation area D2 includes an area D21 indicating a motion type selected by a user operation, an area D22 in which an angle calculated by the calculation unit 12 is displayed, an area D23 in which a maximum value is displayed, an area D24 in which a minimum value is displayed, and the like. In the example in FIG. 4, a flexion motion of the shoulder is selected by the user (area D21). The output processing unit 14 sequentially reflects angles calculated by the calculation unit 12 in the area D22. Further, the output processing unit 14 reflects a maximum value, among the angles sequentially calculated, in the area D23. In the example in FIG. 4, although a hyphen is displayed for the minimum value because it is the first measurement, 0 degrees may be displayed. During measurement after second measurement, the smallest value, among maximum values held as measured values, is output as a minimum value as described above. In a case where the determination unit 13 determines that the posture of the test subject is improper, the output processing unit 14 displays an angle within the area D22 with a red color and displays a line segment superimposed on the video area D1 with a red color.

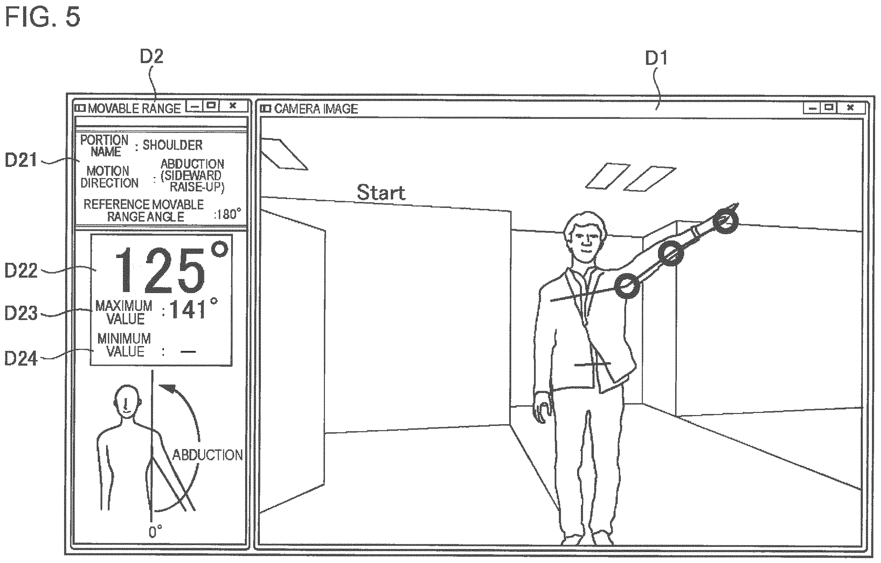

FIG. 5 is a diagram illustrating an example of the output of a display when an ability of an abduction motion of the shoulder is measured. A basic configuration of the display illustrated in FIG. 5 is the same as that in FIG. 4. In the example in FIG. 5, the abduction motion of the shoulder is selected by the user (area D21). The calculation unit 12 calculates an angle as an ability index of the abduction motion by the above-described method. The display in the areas D22, D23 and D24 by the output processing unit 14 is the same as in FIG. 4. In addition, a display in a state corresponding to a determination result regarding whether or not the posture of the test subject is proper is the same as that in FIG. 4.

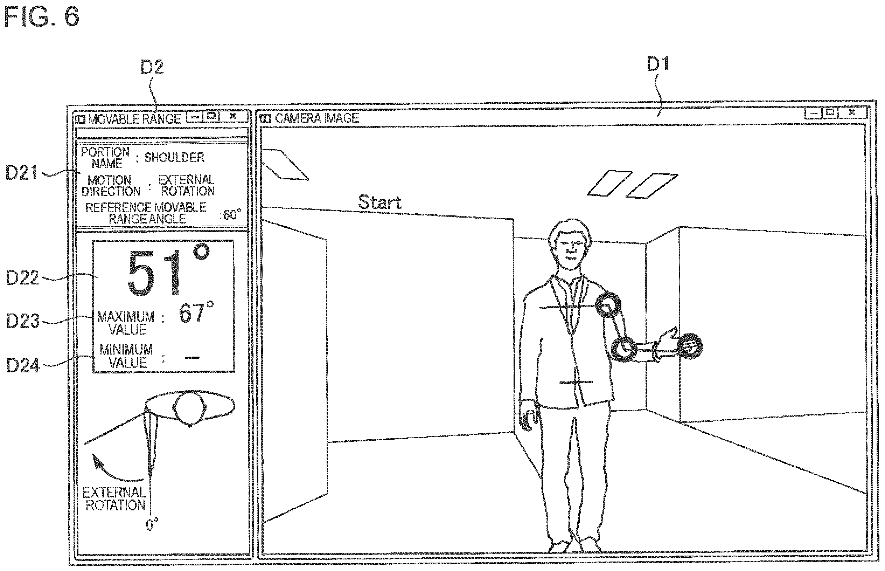

FIG. 6 is a diagram illustrating an example of the output of a display when an ability of an external rotation motion of the shoulder is measured. A basic configuration of the display illustrated in FIG. 6 is the same as that in FIG.

4. In the example in FIG. 6, the external rotation motion of the shoulder is selected by the user (area D21). The calculation unit 12 calculates an angle as an ability index of the external rotation motion by the above-described method. The display in the areas D22, D23 and D24 by the output processing unit 14 is the same as in FIG 4. In addition, a display in a state corresponding to a determination result regarding whether or not the posture of the test subject is proper is the same as that in FIG. 4.

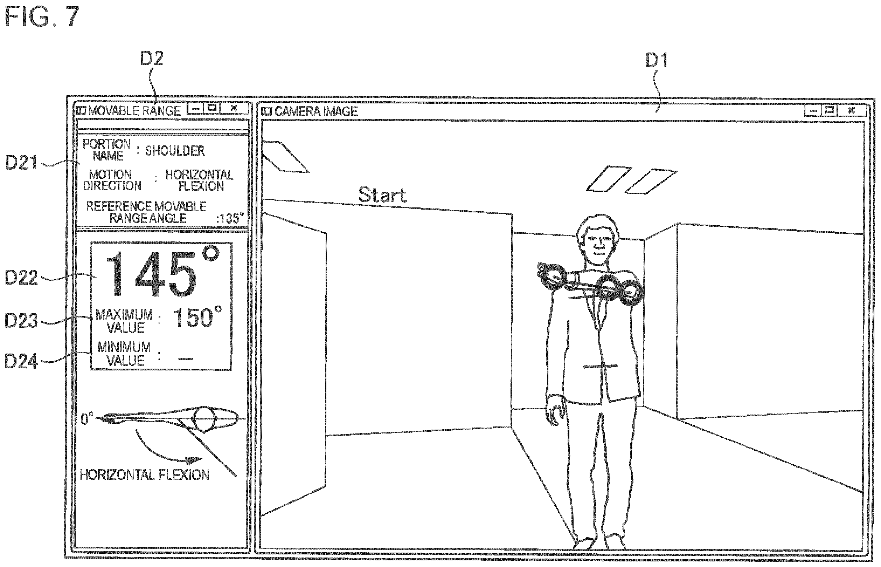

FIG. 7 is a diagram illustrating an example of the output of a display when an ability of a horizontal flexion motion of the shoulder is measured. A basic configuration of the display illustrated in FIG. 7 is the same as that in FIG. 4. In the example in FIG. 7, the horizontal flexion motion of the shoulder is selected by the user (area D21). The calculation unit 12 calculates an angle as an ability index of the horizontal flexion motion by the above-described method. The display in the areas D22, D23 and D24 by the output processing unit 14 is the same as in FIG. 4. In addition, a display in a state corresponding to a determination result regarding whether or not the posture of the test subject is proper is the same as that in FIG. 4.

[Example of Operation/Measurement Method]

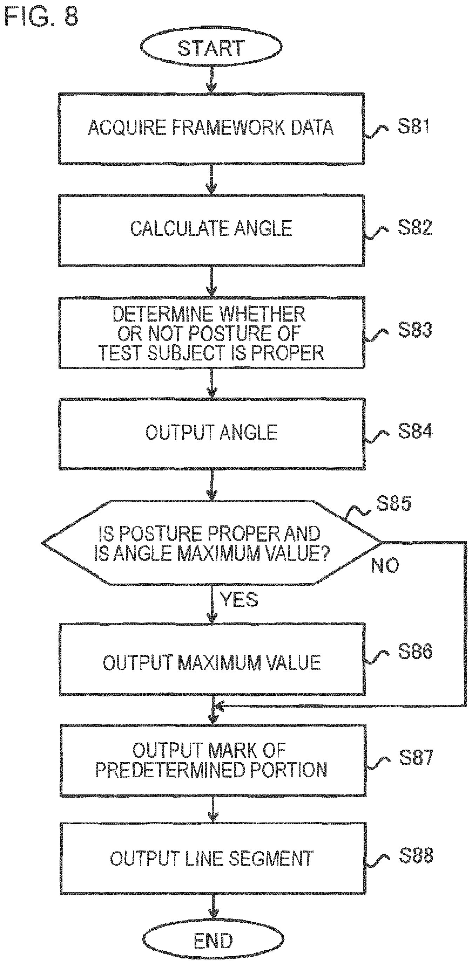

Hereinafter, a measurement method according to the first example embodiment will be described with reference to FIG. 8.

FIG. 8 is a flow chart illustrating an example of the operation of the measurement device 10 according to the first example embodiment. As illustrated in FIG. 8, the measurement method according to the first example embodiment is realized by at least one computer such as the measurement device 10. Processing steps illustrated in FIG. 8 are the same as the processing contents of the above-described processing modules included in the measurement device 10, and thus details of the processing steps will be appropriately omitted.

The measurement device 10 determines one motion type selected by the user among a plurality of motion types determined in advance for the measurement of a shoulder movable range in executing the processing steps illustrated in FIG. 8. For example, the measurement device 10 detects a user operation for selecting one motion type out of a flexion motion, an extension motion, an abduction motion, an adduction motion, an external rotation motion, an internal rotation motion, a horizontal extension motion, and a horizontal flexion motion of the shoulder, and thereby determining the one motion type. In a case where a motion type requiring the measurement of the right and the left is selected, the measurement device 10 automatically determines a direction (left or right) to be measured, or determines the direction by the detection of a user operation.

The measurement device 10 acquires framework data of the test subject who is present within a visual field of the three-dimensional sensor 7 on the basis of information obtained from the three-dimensional sensor 7 (S81). The acquired framework data is positional information on a plurality of predetermined portions of the limbs of the test subject which is used in (S82) and the subsequent steps. The measurement device 10 sequentially acquires the pieces of framework data at predetermined cycles. A method of acquiring the framework data is as described above (data acquisition unit 11).

For example, when the acquisition of the framework data is successful, the measurement device 10 outputs "Start" meaning the start of measurement as illustrated in FIG. 4 and the like. A signal for the start of measurement may be presented by a display, or may be presented by a voice or the like. In addition, the measurement device 10 may output "Ready" indicating a preparation stage until succeeding in the acquisition of the framework data.

The measurement device 10 calculates an angle serving as an ability index of the shoulder of the test subject on the basis of the framework data acquired in (S81) (S82). A method of calculating the angle is as described above, and is determined in advance for each motion type and each direction (right or left) to be measured (calculation unit 12). Accordingly, the measurement device 10 calculates the angle by a measurement method corresponding to a determined motion type and direction (right or left) to be measured. For example, in a case where a flexion motion of the shoulder and the right shoulder are determined, the measurement device 10 calculates an angle on a plane (yz plane) which is perpendicular to the x-axis, the angle being formed by a line segment in the negative direction of the y-axis with the position of the right shoulder as an endpoint and a line segment connecting the position of the right shoulder and the position of the right hand.

The measurement device 10 determines whether or not the posture of the test subject is proper on the basis of the framework data acquired in (S81) (S83). A method of determining the propriety of the posture is as described above, and is determined in advance for each motion type and each direction (right or left) to be measured (determination unit 13). Accordingly, the measurement device 10 determines whether or not the posture of the test subject is proper by a determination method corresponding to a determined motion type and direction (right or left) to be measured. For example, in a case where a flexion motion of the shoulder and the right shoulder are determined, the measurement device 10 detects the body being bent back and forth, the right elbow being bent, and the right shoulder being pulled to the back side to determine that the posture of the test subject is improper. A method of detecting such an improper posture is as described above.

The measurement device 10 outputs the angle calculated in (S82) in a state corresponding to the determination result in (S83) (S84). That is, the measurement device 10 changes a state of the output of the angle in a case where it is determined that the posture of the test subject is proper and a case where it is determined that the posture is improper. For example, the measurement device 10 displays the angle calculated in (S82) in the area D22 as illustrated in the example in FIG 4. The measurement device 10 displays the angle with a black color in a case where it is determined that the posture is proper, and displays the angle with a red color in a case where it is determined that the posture is improper. As described above, a method of outputting the angle by the measurement device 10 is not limited to only a display. In addition, a state of output corresponding to a determination result of the propriety of the posture is not also limited to only coloring.

The measurement device 10 determines whether or not the determination result of (S83) indicates that "the posture is proper" and the angle calculated in (S82) indicates a maximum value (S85). The measurement device 10 determines whether or not the angle calculated in (S82) indicates a maximum angle among angles sequentially calculated for a certain motion type, on the basis of pieces of framework data sequentially acquired at predetermined cycles. In a case where it is determined that the posture is proper and the calculated angle is a maximum value (S85; YES), the measurement device 10 outputs the angle calculated in (S82) as a maximum value (S82) (S86). For example, the measurement device 10 displays the angle calculated in (S82) in the area D23 as illustrated in the example in FIG. 4. On the other hand, the measurement device 10 does not execute (S86) in a case where it is determined that the posture is improper or the calculated angle is not a maximum value (S85; NO). Further, the measurement device 10 excludes the angle calculated in (S82) from the subsequent selection candidates of a maximum value in a case where it is determined that the posture is improper. This is because the angle measured with an improper posture is not set to be an accurate ability index.

The measurement device 10 superimposes a mark on a plurality of predetermined portions of the test subject included in a two-dimensional image obtained from the three-dimensional sensor 7 on the basis of the framework data acquired in (S81) (S87). At this time, the measurement device 10 can convert positional information on a world coordinate system indicated by the framework data into positional information on an image coordinate system of the two-dimensional image obtained from the three-dimensional sensor 7 to determine a position within the image on which the mark is superimposed. In the examples of FIGS. 4 to 7, the measurement device 10 superimposes a mark on the positions of the left shoulder, the left elbow, and the left hand within the image.

Further, the measurement device 10 superimposes a line segment connecting the plurality of predetermined portions on the two-dimensional image on the basis of the framework data acquired in (S81) (S88). At this time, the measurement device 10 can use a correspondence relation between the world coordinate system indicated by the framework data and the image coordinate system of the two-dimensional image. In the examples of FIGS. 4 to 7, the measurement device 10 displays a line segment connecting the left hand, the left elbow, the left shoulder, and the right shoulder and a line segment connecting the left waist and the right waist.

The measurement device 10 can sequentially execute the processing steps illustrated in FIG. 8 whenever a two-dimensional image frame and a depth image (distance image) frame from the three-dimensional sensor 7 are acquired. The processing steps illustrated in FIG. 8 may be executed at intervals longer than a cycle for acquiring the frames. The order of execution of the processing steps in the measurement method of this example embodiment is not limited to the example illustrated in FIG. 8. The order of execution of the processing steps can be changed within a range as long as the change does not affect the content. For example, (S82) and (S83) may be executed in parallel. In addition, the steps (S85) and (S86) and the steps of (S87) and (S88) may be executed in parallel.

[Advantageous Effects of First Example embodiment]

In the first example embodiment, framework data which is positional information on a world coordinate system related to a plurality of predetermined portions of limbs of the test subject is acquired on the basis of information obtained from the three-dimensional sensor 7. A motion type and a target direction (right or left) for measuring a movable range of the shoulder are determined in accordance with a user operation or the like, and an angle serving as an ability index of the motion type is calculated by a method corresponding to the determined motion type and target direction on the basis of the acquired framework data. The calculated angle is output together with a two-dimensional image obtained from the three-dimensional sensor 7. Thereby, the test subject having viewed this output can view a measurement result (angle) while confirming his or her own posture during measurement.

Further, in the first example embodiment, it is determined whether or not the posture of the test subject during the measurement of the ability is proper, in addition to the calculation of an angle serving as an ability index of a certain motion type, and the calculated angle is output in a state corresponding to the determination result. Thereby, it is also possible to provide the propriety of the posture during the measurement to the test subject. Thus, according to the first example embodiment, it is possible to lead the test subject to a correct posture. As a result, even in a state where the test subject does not know a correct posture, it is possible to cause the test subject to correctly perform the measurement of ability by himself or herself. In addition, even when the test subject unconsciously takes an erroneous posture, it is possible to give awareness to the test subject and to accurately measure ability in a correct posture. Further, it is automatically determined whether or not the posture of the test subject is proper, and thus it is possible to eliminate variations in evaluation due to subjectivity and to eliminate a human error such as overlook or erroneous determination.

In addition, in the first example embodiment, a maximum value is output among angles sequentially calculated. Thereby, the test subject can confirm his or her own maximum ability in a certain motion type by viewing the output of the maximum value. In addition, in the first example embodiment, an angle calculated when it is determined that the posture of the test subject is improper is excluded from selection candidates of the maximum value. Thereby, only an ability index measured in a proper posture is set to be a maximum value, and thus it is possible to accurately present the ability of a limb of the test subject. Therefore, according to the first example embodiment, it is possible to accurately measure the ability of a limb of the test subject.

[Second Example embodiment]

In the above-described first example embodiment, framework data (positional information on a predetermined portion) is used, and an angle serving as an ability index of a limb of a test subject is calculated. In a second example embodiment, a distance serving as an ability index of a limb of a test subject is calculated on the basis of a depth image. Hereinafter, with respect to a measurement device 10 and a measurement method according to the second example embodiment, contents different from those in the first example embodiment will be mainly described. In the following description, the same contents as those in the first example embodiment will be appropriately omitted.

In the second example embodiment, an operation detection unit 15 detects a user operation for selecting a flexion motion of a thoracic and lumbar spine. Right and left are not distinguished with each other in the flexion motion of the thoracic and lumbar spine. However, the operation detection unit 15 may detect a user operation for designating whether the test subject faces the right or the left with respect to a three-dimensional sensor 7. In a case where the user operation is detected, a data acquisition unit 11 can determine a direction of the toe or a positional relationship between the toe and a fingertip of the hand on the basis of the designated direction.

The data acquisition unit 11 sequentially acquires a two-dimensional image and a depth image (can also be referred to as a depth information and a distance image) from the three-dimensional sensor 7. Thereby, the data acquisition unit 11 can also be referred to as an image acquisition unit. The depth image indicates depth information (distance from the three-dimensional sensor 7) by a value for each pixel.

The data acquisition unit 11 acquires positional information on a floor and positional information on the toe of the foot of the test subject on the basis of a depth image of the lower half of the body of the test subject which is obtained from the three-dimensional sensor 7. The data acquisition unit 11 can determine a contour of the test subject in the depth image obtained from the three-dimensional sensor 7 by using a pattern matching technique or the like. A two-dimensional image may be further used for the determination of the contour of the test subject. For example, the data acquisition unit 11 can also determine an image region of a person within the two-dimensional image by applying an existing image recognition technique with respect to the two-dimensional image, and can also determine the contour of the test subject and the image region in the depth image by using the determined image region. The data acquisition unit 11 sets the lowermost end of the contour, which is determined in the depth image, on the floor, and acquires positional information on the floor. For example, the acquired positional information on the floor is represented by a y coordinate (pixel position in a vertical direction) of an image coordinate system of the depth image. The "floor" in this determination means a plane on which the test subject stands. Accordingly, the "floor" also includes the surface of a floor of a building, an upper surface of a base, and the like.

Further, the data acquisition unit 11 catches a tip end of the lowermost end of the contour on the x-axis determined in the depth image as the toe of the test subject. The determination of a tip end in which direction out of the right and left on the x-axis may be determined in advance, or may be determined by recognizing the shape of a foot portion (below the ankle). The data acquisition unit 11 acquires positional information on the toe determined on the contour. For example, the acquired positional information on the toe is represented by an x coordinate and a y coordinate of the image coordinate system of the depth image.

In addition, the data acquisition unit 11 acquires positional information on the fingertip of the hand of the test subject which is on the outside of the position of the toe, on the basis of the depth image of the test subject. The data acquisition unit 11 can acquire the depth image of the test subject on the outside of (preceded by) the position of the toe by using contour information on the test subject determined as described above. For example, in a case where the toe faces in the positive direction of the x-axis, the data acquisition unit 11 determines a line segment on the contour having an x coordinate larger than the x coordinate of the position of the toe in the depth image. The data acquisition unit 11 catches the lowermost point of the line segment on the determined contour as the fingertip of the hand of the test subject, and acquires positional information on the fingertip. For example, the acquired positional information on the fingertip of the hand is indicated by the y coordinate (pixel position in a vertical direction) of the image coordinate system of the depth image and a z coordinate (depth) of a world coordinate system.

The calculation unit 12 calculates a distance between the fingertip of the hand of the test subject and the floor as an ability index related to a flexion motion of a thoracic and lumbar spine on the basis of positional information on the floor and positional information on the fingertip of the hand of the test subject which are acquired by the data acquisition unit 11. The calculated distance indicates a distance in the world coordinate system (real world). This calculation method is also based on regulations described in Non-Patent Document 1.

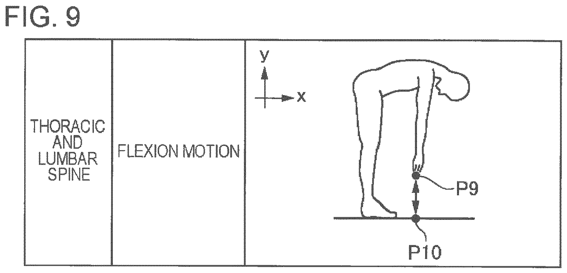

FIG. 9 is a diagram illustrating a method of calculating an ability index related to a flexion motion of a thoracic and lumbar spine. The calculation unit 12 calculates a distance between a fingertip P9 of the test subject and an intersection point P10 between a line segment in the negative direction of the y-axis from the fingertip P9 and the floor. Specifically, the calculation unit 12 calculates the number of pixels PX1 from the fingertip and the floor on the depth image on the basis of the y coordinate (pixel position in a vertical direction) of the fingertip of the hand and the y coordinate (pixel position in a vertical direction) of the floor which are acquired by the data acquisition unit 11. Further, the calculation unit 12 can calculate a distance between the fingertip of the test subject and the floor in the world coordinate system by the following expression by using the calculated number of pixels PX1, a depth DPT of the fingertip P9 of the test subject determined as described above, the number of pixels PX2 which is half the height of the two-dimensional image, and a half (for example, 30 degrees) of a vertical visual field angle of the three-dimensional sensor 7 (visible light camera or the like capturing the two-dimensional image).

$$Distance\ of\ World\ Coordinate\ System= (PX1{\cdot}DPT{\cdot}tan30)/PX2$$

However, a method of calculating a distance between the fingertip of the test subject and the floor is not limited to this expression. For example, PX2 may be set to be the number of pixels which is half the height of the depth image, and the vertical visual field angle may be set to be a vertical visual field angle of the depth sensor. In addition, PX1 may be set to be the number of pixels on the two-dimensional image.

The output processing unit 14 superimposes a mark on the position of the toe of the test subject of the two-dimensional image acquired from the three-dimensional sensor 7 on the basis of the positional information on the toe of the test subject which is acquired by the data acquisition unit 11 while sequentially displaying the two-dimensional image. For example, the output processing unit 14 can align an image region of the test subject on the two-dimensional image and an image region of the test subject which is indicated by the depth image to determine the position of the toe of the test subject on the two-dimensional image by using the positional information on the toe which is acquired by the data acquisition unit 11. This mark also has a purpose of presenting the position of the floor, and thus the output processing unit 14 may superimpose the mark on the position on the floor on the outside of the toe.

The output processing unit 14 fixedly superimposes the mark on images sequentially displayed by the detection of a predetermined event after the superimposition of the mark. The contents of the detected predetermined event are not limited as long as the detected predetermined event is an event for fixing the mark. The detection of a predetermined user operation using the input device 6 may be the predetermined event. In addition, the detection of a predetermined user voice which is input from the input device 6 (microphone) may be the predetermined event. The output processing unit 14 performs coloring based on depth information regarding the test subject on an image region positioned outside the toe of the test subject included in the images sequentially displayed, together with the fixing of the mark. For example, the output processing unit 14 can align the contour of the test subject on the two-dimensional image and the contour of the test subject which is indicated by the depth image to determine an image region on the outside of the toe of the test subject included in the two-dimensional image by using the positional information on the toe of the test subject which is acquired by the data acquisition unit 11. The output processing unit 14 can superimpose depth information (pixel value) on the corresponding region on the depth image on the determined image region of the two-dimensional image to perform coloring based on the depth information on the test subject.

The output processing unit 14 may output a minimum value among distances sequentially calculated by the calculation unit 12. For example, the output processing unit 14 can also exclude a distance calculated when the determination unit 13 determines that the posture of the test subject is improper from selection candidates of the minimum value while outputting the minimum value of the distance. In addition, the output processing unit 14 can also hold a finally determined minimum value as a measured value for each measurement, and can also output the largest value, among measured values held by that time, as a maximum value. In a case where the output minimum value is larger than the output maximum value during each measurement after second measurement, the output processing unit 14 may output the minimum value as a maximum value.

The determination unit 13 determines whether or not the posture of the test subject is proper on conditions determined in advance with respect to the flexion motion of the thoracic and lumbar spine. For example, the determination unit 13 detects the knee being bent, the waist being pulled, and the like on the basis of framework data acquired by the data acquisition unit 11 to determine that the posture of the test subject is improper. For example, the determination unit 13 confirms a deviation on the x-axis of each portion on the basis of framework data of the right waist, the right knee, and the right foot. The determination unit 13 determines that the posture of the test subject is improper in a case where only the x coordinate of the right waist or the right knee exceeds a predetermined range with the x coordinate of the right foot as a reference.

[Example of Output]

Next, a specific example of the output of a display by the output processing unit 14 according to the second example embodiment will be described.

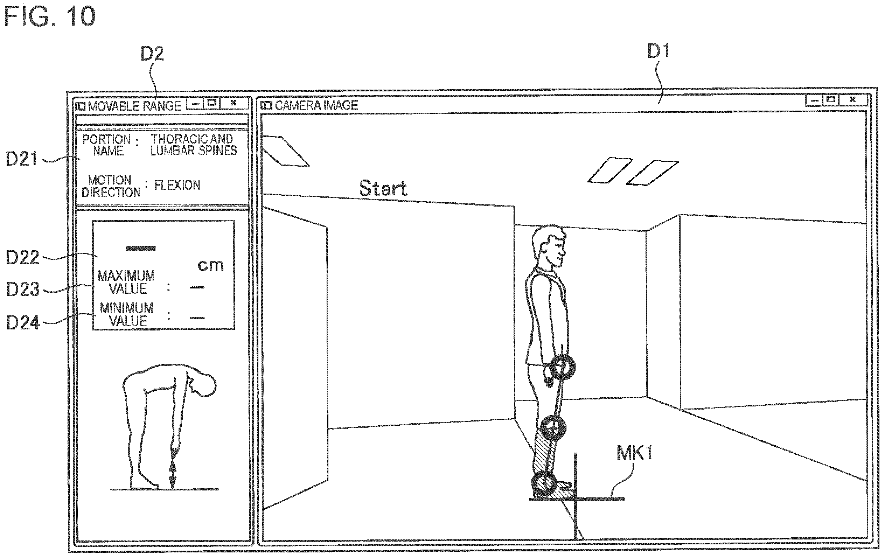

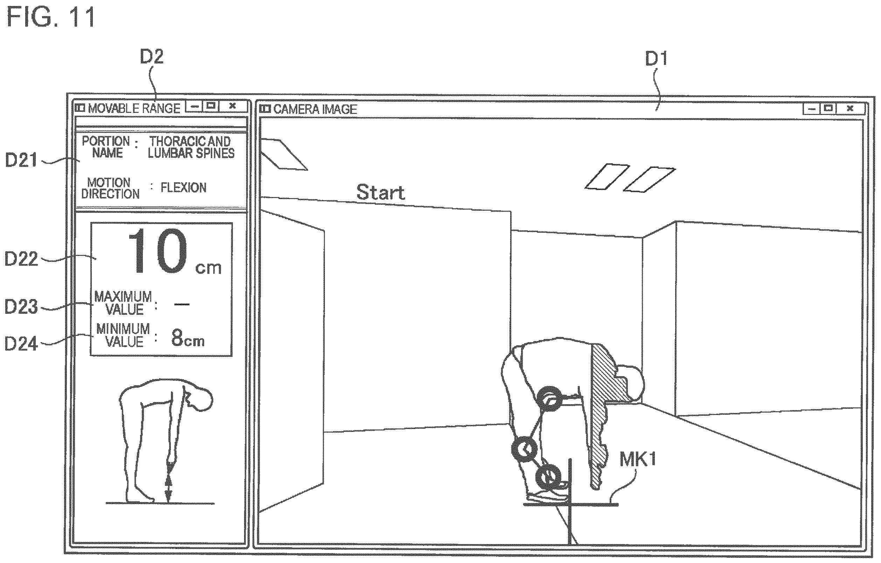

FIGS. 10 and 11 are diagrams illustrating an example of the output of a display when an ability of a flexion motion of a thoracic and lumbar spine is measured. The arrangement of displays areas of a video area D1, an explanation area D2, and the like is the same as those in FIG. 4 and the like. The output processing unit 14 displays the flexion motion of the thoracic and lumbar spine being selected by the user in the area D21, displays distances calculated by the calculation unit 12 in the area D22, and displays a minimum value of the distances in an area D24. However, since measurement has not been started at a point in time in FIG. 10, a value is not displayed (display of a hyphen) in areas D22 and D24. In the examples of FIGS. 10 and 11, a maximum value is displayed as a hyphen in the area D24, but the largest value among minimum values held as measured values may be output as a maximum value as described above.

The output processing unit 14 superimposes a mark on the positions of the right foot, the right knee, and the right waist on a two-dimensional image while displaying the two-dimensional image in the video area D1 at any time, similar to FIG. 4 and the like. However, in a case where the test subject faces in a direction opposite to the three-dimensional sensor 7, the output processing unit 14 superimposes a mark on the positions of the left foot, the left knee, and the left waist on the two-dimensional image. A method of displaying the mark on each portion is the same as that in the first example embodiment. In the second example embodiment, the output processing unit 14 further superimposes a mark on the position of the toe of the test subject on the two-dimensional image. In the examples of FIGS. 10 and 11, a mark MK1 being a cross pattern is displayed. The mark indicates the floor by the horizontal line thereof. When the output processing unit 14 detects a predetermined event, the output processing unit fixes the display position of the mark MK1 at the position at that time. The output processing unit 14 fixes the display position of the mark MK1, and then performs coloring based on the depth information regarding the test subject on an image region positioned on the outside of the toe of the test subject included in the two-dimensional image, as illustrated in FIG. 11.

[Example of Operation/Measurement Method]

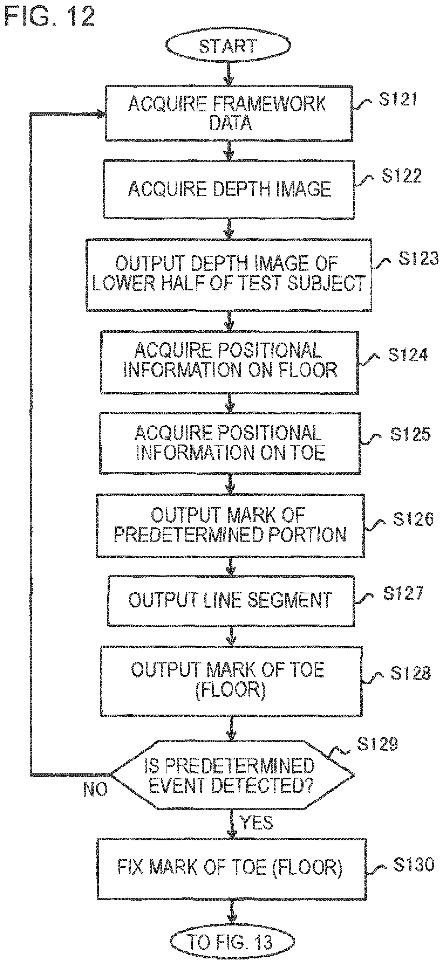

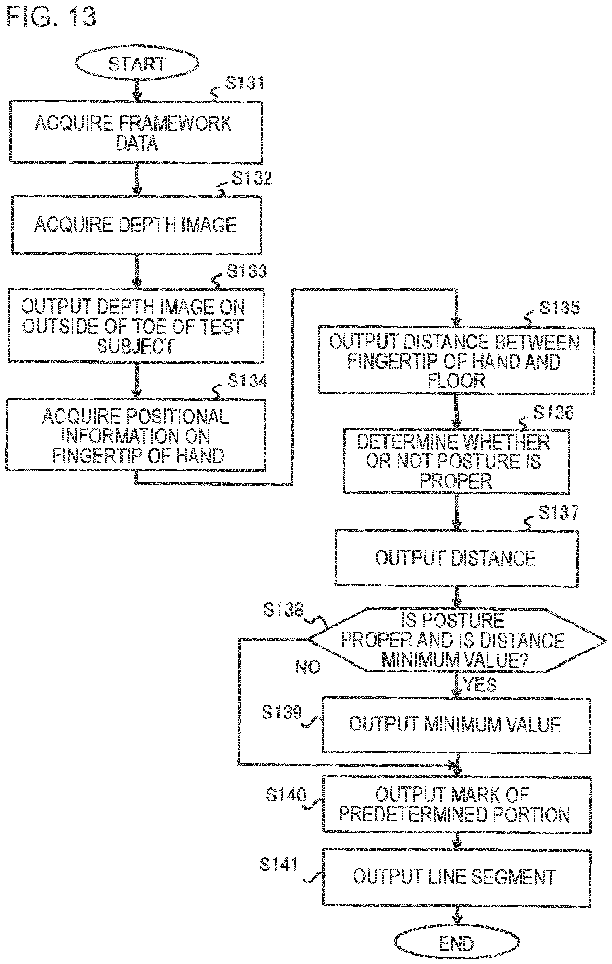

Hereinafter, a measurement method according to the first example embodiment will be described with reference to FIGS. 12 and 13.

FIGS. 12 and 13 are flow charts illustrating an example of the operation of the measurement device 10 according to the second example embodiment. As illustrated in FIGS. 12 and 13, a measurement method according to the second example embodiment is executed by at least one computer such as the measurement device 10. Processing steps illustrated in FIGS. 12 and 13 are the same as the processing contents of the above-described processing modules included in the measurement device 10, and thus details of the processing steps will be appropriately omitted.

The measurement device 10 detects a user operation for selecting a flexion motion of a thoracic and lumbar spine in executing the processing steps illustrated in FIGS. 12 and 13. The measurement device 10 executes the processing steps illustrated in FIG. 8 in a case where a motion type for measuring a movable range of the shoulder is selected.

The measurement device 10 acquires pieces of framework data of the test subject which is present within a visual field of the three-dimensional sensor 7 on the basis of information obtained from the three-dimensional sensor 7 (S121). The acquired pieces of framework data are pieces of positional information on a plurality of predetermined portions of limbs of the test subject which is used in (S122) and the subsequent steps. The measurement device 10 sequentially acquires the pieces of framework data at predetermined cycles. A method of acquiring the framework data is as described in the first example embodiment (data acquisition unit 11).

Further, the measurement device 10 acquires depth images (S122). The measurement device 10 sequentially acquires the depth images at predetermined cycles.

The measurement device 10 outputs a depth image of the lower half of the test subject on the basis of the depth images acquired in (S122) (S123). Specifically, the measurement device 10 superimposes the depth image of the lower half of the test subject among the depth images acquired in (S122) on an image region indicating the lower half of the test subject on a two-dimensional image acquired from the three-dimensional sensor 7 while displaying the two-dimensional image. For example, the measurement device 10 can determine an image region of the lower half of the test subject in the two-dimensional image by using an existing image recognition technique. Further, the measurement device 10 can determine a contour and an image region of the lower half of the test subject in the depth image by using an existing pattern matching technique or the like.

The measurement device 10 acquires positional information on the floor on the basis of the depth image of the lower half of the test subject which is determined as described above (S124). Specifically, the measurement device 10 sets the lowermost end of the contour of the lower half of the test subject to be the floor, and acquires positional information on the floor. The measurement device 10 can acquire a y coordinate (pixel position) of an image coordinate system as the positional information on the floor.

Further, the measurement device 10 acquires positional information on the toe of the test subject on the basis of the depth image of the lower half of the test subject (S125). Specifically, the measurement device 10 catches a tip end on the x-axis of the lowermost end determined in (S124) as the toe of the test subject. The measurement device 10 can acquire an x coordinate and a y coordinate (pixel position) of the image coordinate system as the positional information on the toe.

The measurement device 10 superimposes a mark on a plurality of predetermined portions of the test subject included in the two-dimensional image acquired from the three-dimensional sensor 7 on the basis of the framework data acquired in (S121) (S126). A method of displaying the mark of the predetermined portion is as described in the first example embodiment. In the example in FIG. 10, the measurement device 10 superimposes a mark on the positions of the right foot, the right knee, and the right waist within the image.

Further, the measurement device 10 superimposes a line segment connecting the plurality of predetermined portions on the two-dimensional image on the basis of the framework data acquired in (S121) (S127). A method of displaying the line segment is also as described in the first example embodiment. In the example in FIG. 10, a line segment connecting the right waist, the right knee, and the right foot is displayed.

When the measurement device 10 detects a predetermined event such as a predetermined user operation using the input device 6 (S129; YES), the measurement device fixes a display position of the mark (mark of the toe), which is superimposed on the position of the toe, on the two-dimensional image (S131). At this time, the measurement device 10 holds the pieces of latest positional information on the floor and the toe which are acquired in (S124) and (S125). The measurement device 10 repeatedly executes steps (S121) to (S128) while the predetermined event is not detected (S129; NO).

The measurement device 10 newly acquires framework data and a depth image in the next cycle after the predetermined event is detected (S129 in FIG. 12; YES) (S131) (S132).

The measurement device 10 outputs a depth image on the outside of the toe of the test subject on the basis of the depth image acquired in (S132) and the positional information on the toe which is held by the operation in FIG. 12 (S133). Specifically, the measurement device 10 superimposes a depth image of a portion on the outside of the toe of the test subject, among the depth images acquired in (S132), on an image region indicating the outside of the toe of the test subject on the two-dimensional image acquired from the three-dimensional sensor 7 while displaying the two-dimensional image. Since positional information on the image coordinate system is held as the positional information on the toe, an image region of the entire test subject is determined in each of the two-dimensional image and the depth image, and thus it is possible to determine the image region indicating the outside of the toe of the test subject in each image.

The measurement device 10 catches the lowermost end in the depth image on the outside of the toe of the test subject which is output in (S133) as the fingertip of the hand of the test subject, and acquires positional information on the lowermost end as positional information on the fingertip of the hand of the test subject (S134). As the positional information on the fingertip of the hand of the test subject, an x coordinate and a y coordinate of an image coordinate system and a z coordinate (depth) in a world coordinate system can be acquired.

The measurement device 10 calculates a distance between the fingertip of the hand of the test subject and the floor on the basis of the positional information on the floor which is held by the operation in FIG. 12 and the positional information on the fingertip acquired in (S134) (S135). The measurement device 10 calculates a distance in the world coordinate system. A method of calculating this distance is as described above (calculation unit 12).

The measurement device 10 determines whether or not the posture of the test subject is proper on the basis of the framework data acquired in (S131) (S136). The measurement device 10 detects the knee being bent, the waist being pulled, and the like with respect to the flexion motion of the thoracic and lumbar spine to determine that the posture of the posture is improper. A method of detecting an improper posture is as described above (determination unit 13).

The measurement device 10 outputs the distance calculated in (S135) in a state corresponding to the determination result in (S136) (S137). That is, the measurement device 10 changes a state of the output of the distance in a case where it is determined that the posture of the test subject is proper and a case where the posture is improper. For example, the measurement device 10 displays the distance calculated in (S135) in an area D22 as illustrated in the example in FIG. 11. The measurement device 10 displays the distance with a black color in a case where it is determined that the posture is proper, and displays the distance with a red color in a case where it is determined that the posture is improper. As described above, a method of outputting a distance by the measurement device 10 is not limited to only a display. In addition, a state of output corresponding to a determination result of the propriety of the posture is not also limited to only coloring.

The measurement device 10 determines whether or not the determination result of (S13 6) indicates that "the posture is proper" and the distance calculated in (S135) indicates a minimum value (S138). The measurement device 10 determines whether or not the distance calculated in (S135) indicates the smallest distance among distances sequentially calculated on the basis of the depth images sequentially acquired at predetermined cycles. The measurement device 10 outputs the distance calculated in (S135) as the minimum value in a case where it is determined that the posture is proper and the calculated distance indicates a minimum value (S138; YES)

(S139). For example, the measurement device 10 displays the distance calculated in (S135) in an area D24 as illustrated in the example in FIG. 11. On the other hand, the measurement device 10 does not execute (S139) in a case where it is determined that the posture is improper or the calculated distance is not a minimum value (S138; NO). Further, the measurement device 10 excludes the distance calculated in (S135) from the subsequent selection candidates of a minimum value in a case where it is determined that the posture is improper. This is because the distance measured with an improper posture is not set to be an accurate ability index.

The measurement device 10 superimposes a mark on a plurality of predetermined portions of the test subject included in the two-dimensional image obtained from the three-dimensional sensor 7 on the basis of the framework data acquired in (S131) (S140). A method of displaying the mark on the predetermined portion is as described in the first example embodiment.

Further, the measurement device 10 superimposes a line segment connecting the plurality of predetermined portions on the two-dimensional image on the basis of the framework data acquired in (S131) (S141). A method of displaying the line segment is as described in the first example embodiment.

The measurement device 10 can sequentially execute the processing steps illustrated in FIG. 13 whenever a two-dimensional image frame and a depth image (distance image) frame are acquired from the three-dimensional sensor 7 after the predetermined event is detected. The processing steps illustrated in FIG. 13 may be executed at intervals longer than a cycle for acquiring the frames. The order of execution of the processing steps in the measurement method of this example embodiment is not limited to the examples illustrated in FIGS. 12 and 13. The order of execution of the processing steps can be changed within a range as long as the change does not affect the content. For example, (S122) may be executed prior to (S121). In addition, (S126) and (S127) may be executed at any stage as long as the steps are executed after (S121).

[Advantageous Effects of Second EXAMPLE EMBODIMENT]

In the second example embodiment, positional information on the toe of the foot of the test subject and positional information on the floor are acquired on the basis of a depth image acquired from the three-dimensional sensor 7, and a mark is displayed at the position of the toe of the test subject included in a two-dimensional image acquired from the three-dimensional sensor 7. Positional information on the right foot and the left foot in a world coordinate system can be acquired as framework data. However, even a foot portion (below the ankle) has a certain degree of width, and thus the position thereof is different in units of 10 centimeters in the toe and the center of the foot portion. According to the second example embodiment, positional information is acquired from a contour and an image region of the test subject which are determined on the depth image, and thus it is possible to more accurately obtain the positions of the floor and the toe.

Further, in the second example embodiment, the positions of the toe and the floor are decided by the detection of a predetermined event, and a contour and an image region of the test subject which are on the outside of the toe in the depth image are determined on the basis of positional information on the decided toe, and the lowermost end thereof is determined to be the position of the fingertip of the hand of the test subject. A distance (the number of pixels) between the fingertip of the hand of the test subject and the floor in an image coordinate system of the depth image is calculated, and the distance of the image coordinate system is converted into a distance of a world coordinate system. Therefore, according to the second example embodiment, it is possible to calculate a distance serving as an ability index of a flexion motion of a thoracic and lumbar spine with a higher level of accuracy than in measurement using framework data.

In addition, in the second example embodiment, coloring based on depth information is performed on an image region positioned outside the toe of the test subject in a displayed two-dimensional image after the positions of the toe and the floor are decided by the detection of a predetermined event. Thereby, it is possible to easily recognize which portion of the test subject included in the two-dimensional image is recognized as the fingertip of the hand, and to confirm that the measurement device 10 is normally operating.

[Third EXAMPLE EMBODIMENT]

In the above-described second example embodiment, a distance between the fingertip of the hand of a test subject and a floor is calculated as an ability index of a limb of the test subject on the basis of a depth image. In the third example embodiment, a distance between the positions of the fingertip of the hand of the test subject at different points in time is calculated as an ability index of a limb of the test subject on the basis of the depth image. Hereinafter, with respect to a measurement device 10 and a measurement method according to a third example embodiment will be described, contents different from those in the above-described example embodiments will be mainly described. In the following description, the same contents as those in the above-described example embodiments will be appropriately omitted.

In the third example embodiment, an operation detection unit 15 detects a user operation for selecting a functional reach test (hereinafter, simply referred to as FRT). FRT is a method well known as a method of evaluating a balancing function of a person, and is a method which is often used in a clinical site in order to obtain an index for predicting dangerousness of falling. Right and left are not distinguished with each other in FRT. However, the operation detection unit 15 may detect a user operation for designating whether the test subject faces the right or the left with respect to a three-dimensional sensor 7. In a case where the user operation is detected, a data acquisition unit 11 can determine whether the fingertip of the hand is positioned at the right end or the left end in a contour of the test subject in the depth image, on the basis of the designated direction.

The data acquisition unit 11 sequentially acquires depth images from the three-dimensional sensor 7. The data acquisition unit 11 sequentially acquires depth images of the whole body of the test subject, and sequentially acquires pieces of positional information on the fingertip of the hand of the test subject on the basis of the depth images. As described above, the data acquisition unit 11 can determine the contour of the test subject in the depth image obtained from the three-dimensional sensor 7 by using a pattern matching technique or the like. The data acquisition unit 11 can catch a point having the largest x coordinate in the determined contour of the test subject as the fingertip of the hand of the test subject to acquire positional information (pixel position) on the fingertip in an image coordinate system of the depth image. Since framework data is not used with respect to FRT, the data acquisition unit 11 may not acquire framework data in a case where the measurement device 10 supports only FRT.

The data acquisition unit 11 holds positional information on the fingertip of the hand, which is acquired during the detection of a predetermined event, by the detection. The contents of the detected predetermined event are not limited as long as the detected predetermined event is an event for fixing a reference position of the fingertip. The detection of a predetermined user operation using an input device 6 may be the predetermined event. In addition, the detection of a predetermined user voice which is input from the input device 6 (microphone) may be the predetermined event. For example, the data acquisition unit 11 holds a contour pattern in advance in a state where a person normally stands and pushes out the arm straight ahead, and detects this contour pattern from the depth image as the detection of the predetermined event.

The calculation unit 12 calculates a distance in the x-axis direction between the position of the fingertip of the hand corresponding to the held positional information and the position of the fingertip of the hand corresponding to newly acquired positional information, on the basis of the positional information held by the detection of the predetermined event and the newly held positional information. The calculated distance is a distance of a world coordinate system, and serves an ability index of FRT. Specifically, the calculation unit 12 calculates a distance (the number of pixels) between the fingertips of the hands in the image coordinate system of the depth image on the basis of the two pieces of positional information, and converts the distance into a distance of the world coordinate system by the same method as in the second example embodiment.

The determination unit 13 determines whether or not the posture of the test subject is proper on a condition which is determined in advance with respect to FRT. For example, the determination unit 13 detects that the position of the fingertip of the hand exceeds a predetermined range in the y-axis direction, and the like on the basis of the held positional information and the newly acquired positional information to determine that the posture of the test subject is improper. In this case, the determination unit 13 determines that the posture of the test subject is improper when a difference between a y coordinate of the held positional information and a y coordinate of the newly acquired positional information exceeds a predetermined threshold value.

[Example of Output]

Next, a specific example of the output of a display by the output processing unit 14 according to the third example embodiment will be described.

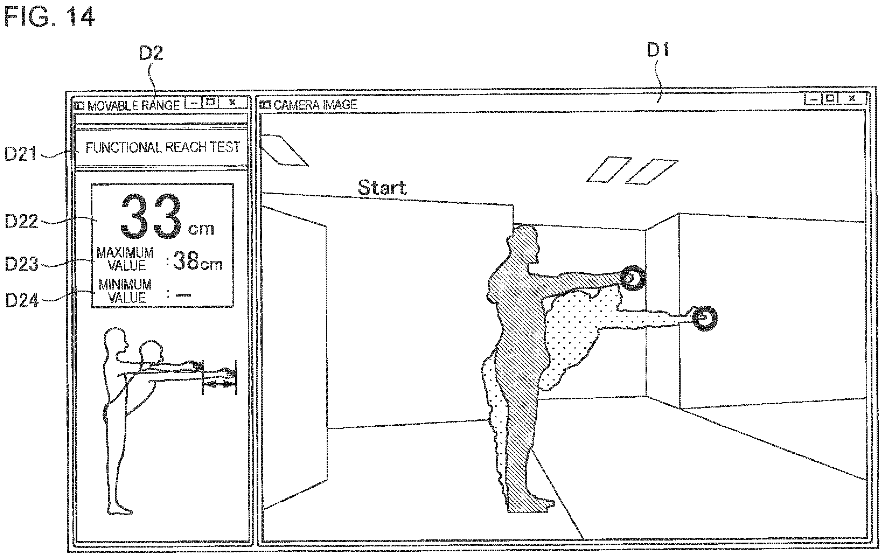

FIG. 14 is a diagram lustrating an example of the output of a display when an ability of the functional reach test is measured. The arrangement of displays areas of a video area D1, an explanation area D2, and the like is the same as those in FIG. 4 and the like. The output processing unit 14 displays FRT being selected by the user in an area D21, displays distances calculated by the calculation unit 12 in an area D22, and displays a maximum value of the distances in an area D23. Meanwhile, also in FRT, the minimum value may be displayed similar to the examples in FIG. 4 and the like.

The output processing unit 14 superimposes a mark on the position of the fingertip on a two-dimensional image while displaying the two-dimensional image in the video area D1 at any time, similar to FIG. 4 and the like. For example, the output processing unit 14 can align an image region of the test subject on the two-dimensional image and an image region of the test subject which is indicated by the depth image to determine the position of the fingertip of the hand of the test subject on the two-dimensional image by using the positional information on the fingertip of the hand which is acquired by the data acquisition unit 11. When a predetermined event is detected, the output processing unit 14 fixedly superimposes a contour and an image region of the test subject, which are determined on the depth image during the detection, on the two-dimensional image, and also fixes a mark indicating the position of the fingertip of the hand on the two-dimensional image. Further, the output processing unit 14 superimposes a mark on a newly acquired position of the fingertip of the hand while also superimposing the contour and the image region of the test subject which are determined on the depth image after the detection of the predetermined event on the two-dimensional image. At this time, the depth image of the test subject which is fixed on the two-dimensional image and the depth image of the test subject which is superimposed on the two-dimensional image are displayed so as to be distinguished from each other by changing the colors thereof.

[Example of Operation/Measurement Method]

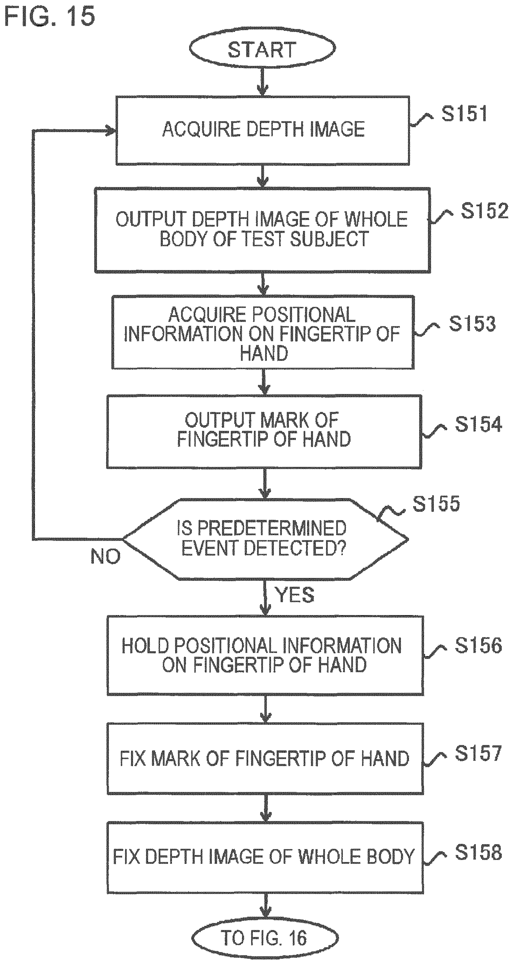

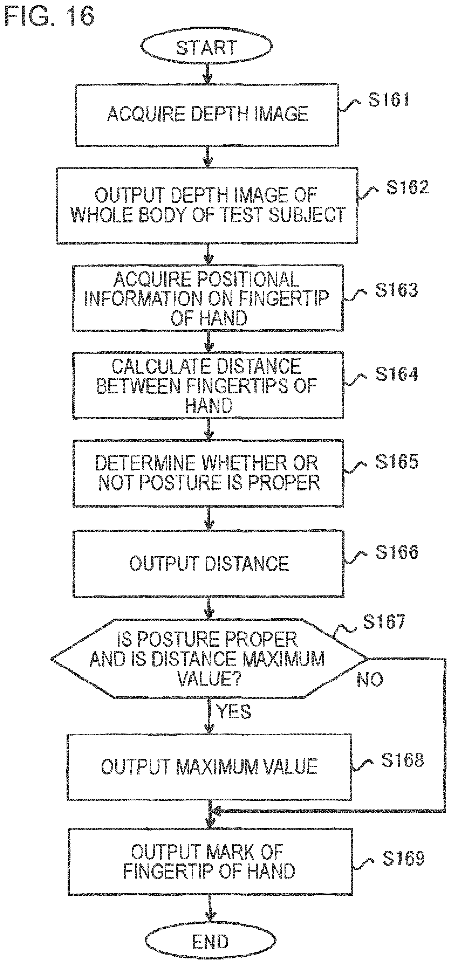

Hereinafter, a measurement method according to the third example embodiment will be described with reference to FIGS. 15 and 16.

FIGS. 15 and 16 are flow charts illustrating an example of the operation of the measurement device 10 according to the third example embodiment. As illustrated in FIGS. 15 and 16, a measurement method according to the third example embodiment is executed by at least one computer such as the measurement device 10. Processing steps illustrated in FIGS. 15 and 16 are the same as the processing contents of the above-described processing modules included in the measurement device 10, and thus details of the processing steps will be appropriately omitted.

The measurement device 10 detects a user operation for selecting FRT in executing the processing steps illustrated in FIGS. 15 and 16. The measurement device 10 executes the processing steps illustrated in FIG. 15 when the user operation for selecting FRT is detected.

The measurement device 10 acquires depth images from the three-dimensional sensor 7 (S151). The measurement device 10 sequentially acquires the depth images at predetermined cycles.

The measurement device 10 outputs a depth image of the whole body of the test subject on the basis of the depth images acquired in (S151) (S152). Specifically, the measurement device 10 superimposes the depth image of the whole body of the test subject, among the depth images acquired in (S151), on an image region indicating the whole body of the test subject on a two-dimensional image obtained from the three-dimensional sensor 7 while displaying the two-dimensional image. For example, the measurement device 10 can determine the image region of the whole body of the test subject in the two-dimensional image by using an existing image recognition technique. Further, the measurement device 10 can determine a contour and an image region of the whole body of the test subject in the depth image by using existing pattern matching technique or the like.

The measurement device 10 acquires positional information on the fingertip of the hand of the test subject on the basis of the depth image of the whole body of the test subject which is determined as described above (S153). Specifically, the measurement device 10 can catch a point having the largest x coordinate in the depth image of the whole body of the test subject as the fingertip of the hand of the subject to acquire positional information (pixel position) on the fingertip in an image coordinate system of the depth image.

The measurement device 10 superimposes a mark on the position of the fingertip of the hand of the test subject in the displayed two-dimensional image (S154). The measurement device 10 can align an image region of the test subject on the two-dimensional image and an image region of the test subject which is indicated by the depth image to determine the position of the fingertip of the hand of the test subject on the two-dimensional image by using the positional information on the fingertip of the hand which is acquired in (S153).

When the measurement device 10 detects a predetermined event such as a predetermined user operation using the input device 6 (S155; YES), the measurement device holds the latest positional information on the fingertip of the hand which is acquired in (S153) (S156). Further, the measurement device 10 fixes a display position of the mark (mark of the fingertip), which is superimposed on the position of the fingertip of the hand, on the two-dimensional image (S157). In addition, the measurement device 10 fixes a display position on the two-dimensional image of the depth image of the whole body of the test subject which is output in (S152) during the detection of the predetermined event (S158). The measurement device 10 repeatedly executes steps (S151) to (S154) while the predetermined event is not detected (S155; NO).

The measurement device 10 newly acquires a depth image in the next cycle after the predetermined event is detected (S155 in FIG. 15; YES) (S161).

The measurement device 10 executes (S162) and (S163) on the basis of the newly acquired depth image. Step (S162) is the same as (S152) illustrated in FIG. 15, and step (S163) is the same as (S153) illustrated in FIG. 15.

The measurement device 10 calculates a distance in the x-axis direction between the positions of the fingertip of the hand which correspond to the pieces of positional information on the basis of the positional information held in (S156) and the positional information newly acquired in (S163) (S164). The measurement device 10 calculates a distance in a world coordinate system. A method of calculating this distance is as described above (calculation unit 12).

The measurement device 10 determines whether or not the posture of the test subject is proper on the basis of the positional information held in (S156) and the positional information newly acquired in (S163) (S165). The measurement device 10 detects that the position of the fingertip of the hand exceeds a predetermined range in the y-axis direction with respect to FRT, and thus the measurement device determines that the posture of the test subject is improper.

The measurement device 10 outputs the distance calculated in (S164) in a state corresponding to the determination result in (S165) (S166). That is, the measurement device 10 changes a state of the output of the distance in a case where it is determined that the posture of the test subject is proper and a case where it is determined that the posture is improper. For example, the measurement device 10 displays the distance calculated in (S164) in the area D22 as illustrated in the example in FIG 14. The measurement device 10 displays the distance with a black color in a case where it is determined that the posture is proper, and displays the distance with a red color in a case where it is determined that the posture is improper. As described above, a method of outputting the distance by the measurement device 10 is not limited to only a display. In addition, a state of output corresponding to a determination result of the propriety of the posture is not also limited to only coloring.

The measurement device 10 determines whether or not the determination result of (S165) indicates that "the posture is proper" and the distance calculated in (S164) indicates a maximum value (S167). The measurement device 10 determines whether or not the distance calculated in (S164) indicates the largest distance among distances sequentially calculated on the basis of the depth images sequentially calculated at predetermined cycles. In a case where it is determined that the posture is proper and the calculated distance indicates a maximum value (S167; YES), the measurement device 10 outputs the distance calculated in (S164) as a maximum value (S168). For example, the measurement device 10 displays the distance calculated in (S164) in the area D23 as illustrated in the example in FIG. 14. On the other hand, the measurement device 10 does not execute (S168) in a case where it is determined that the posture is improper or the calculated distance is not a maximum value (S167; NO). Further, the measurement device 10 excludes the distance calculated in (S164) from the subsequent selection candidates of a maximum value in a case where it is determined that the posture is improper. This is because the distance measured with an improper posture is not set to be an accurate ability index.

The measurement device 10 superimposes a mark on the position of the fingertip of the hand of the test subject in the displayed two-dimensional image (S169). Step (S169) is the same as (S154) illustrated in FIG 15.

The measurement device 10 can sequentially execute the processing steps illustrated in FIG. 16 whenever a two-dimensional image frame and a depth image (distance image) frame from the three-dimensional sensor 7 are acquired after the operation in FIG. 15 is executed. The processing steps illustrated in FIG. 16 may be performed at intervals longer than a cycle for acquiring the frames. The order of execution of the processing steps in the measurement method according to this example embodiment is not limited to the examples illustrated in FIGS. 15 and 16. The order of execution of the processing steps can be changed within a range as long as the change does not affect the content. For example, (S157) and (S158) may be executed in parallel or in the reverse order. In addition, (S165) may be executed prior to (S164).

[Advantageous Effects of Third Example embodiment]

In the third example embodiment, positional information on the fingertip of the hand of the test subject is acquired on the basis of a depth image obtained from the three-dimensional sensor 7, and a distance between positions respectively corresponding to positional information during the detection of a predetermined event and positional information acquired thereafter regarding the fingertip of the hand of the test subject is calculated as an ability index of FRT. In this manner, according to the third example embodiment, a distance between the positions of the fingertip of the hand at two certain points in time is calculated on the basis of the depth image, and thus it is possible to calculate the distance with a high level of accuracy than in a case where framework data is used.

In addition, in the third example embodiment, the position of the fingertip of the hand which is a reference of the calculated distance is decided by the detection of a predetermined event, and a mark is fixedly displayed on the decided position in a two-dimensional image. In the third example embodiment, after the predetermined event is detected, a mark indicating the latest position of the fingertip of the hand is further displayed together with the mark which is fixedly displayed. Therefore, according to the third example embodiment, it is possible to provide a measurement process of FRT to the test subject so that the test subject easily understand the measurement process.

[Fourth EXAMPLE EMBODIMENT]

In a fourth example embodiment, an angle serving as an ability index of a right rotation motion or a left rotation motion of a neck is calculated on the basis of the direction of a face and opening angles of both shoulders. Hereinafter, with respect to a measurement device 10 and a measurement method according to the fourth example embodiment, contents different from those in the above-described example embodiments will be mainly described. In the following description, the same contents as those in the above-described example embodiments will be appropriately omitted.

[Processing Configuration]

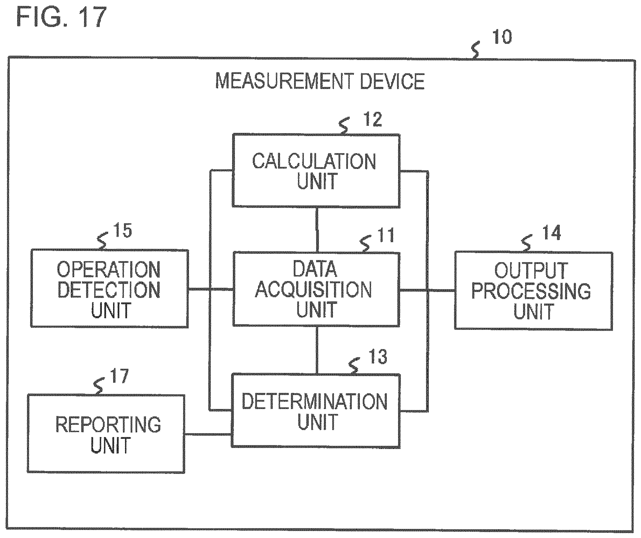

FIG. 17 is a schematic diagram illustrating an example of a processing configuration of the measurement device 10 according to the fourth example embodiment. As illustrated in FIG. 17, the measurement device 10 according to the fourth example embodiment further includes a reporting unit 17 in addition to the configurations of the above-described example embodiments. The reporting unit 17 is also realized similar to other processing modules.

In the fourth example embodiment, an operation detection unit 15 detects a user operation for selecting a turning motion for the measurement of a neck movable range. This turning motion includes a left turning motion and a right turning motion. Accordingly, the operation detection unit 15 may detect an operation of selecting which one of the left and the right is measured, with respect to the turning motion.

A data acquisition unit 11 acquires direction information on the face of the test subject positioned within a visual field of a three-dimensional sensor 7, positional information on both shoulders, and depth information on the test subject, on the basis of information obtained from the three-dimensional sensor 7. The data acquisition unit 11 can acquire the positional information on both shoulders as framework data by the same method as in the first example embodiment. In addition, the data acquisition unit 11 can recognize a person's face from a two-dimensional image and a depth image which are obtained from the three-dimensional sensor 7 by using an existing image recognition technique, and can determine the direction of the face of the person from a positional relationship between portions of the recognized face. Various existing image recognition methods can be used as a method of recognizing the face of the person and the portions within the face. The direction information on the face which is acquired by the data acquisition unit 11 is represented by an angle from the z-axis. The acquired depth information on the test subject is information based on the depth image, and indicates a depth of a certain point of a limb of the test subject, such as the center of the face, or a depth of the central position of a plurality of points of the limb of the test subject.

Incidentally, there is a limitation in the determination of the direction of the face of the person based on the information obtained from the three-dimensional sensor 7. In a case where the two-dimensional image obtained from the three-dimensional sensor 7 includes only a side face, the face of the person may not be recognized. Accordingly, in a case where the direction information on the face is obtained only with respect to, for example, 45 degrees, it is not possible to measure a movable range of the neck. Consequently, in this example embodiment, the test subject is made to obliquely face the three-dimensional sensor 7, so that an angle serving as an ability index of the rotation motion of the neck is calculated from the opening angle of the shoulder and the direction information on the face which is obtained by the data acquisition unit 11.

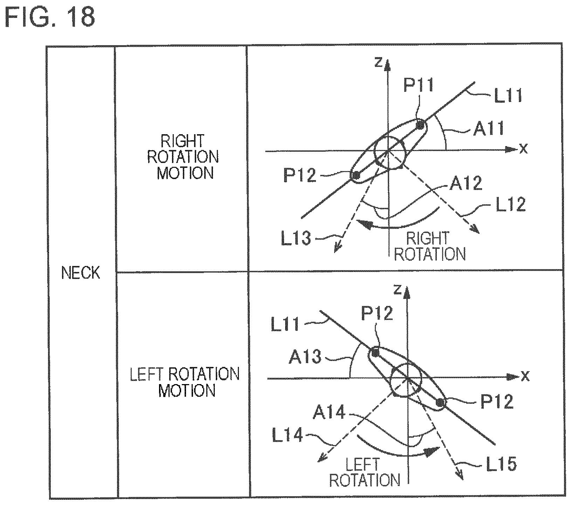

Specifically, on the basis of the positional information on both shoulders which is acquired by the data acquisition unit 11, as an opening angle of the shoulder, a calculation unit 12 calculates an angle that is formed by a line segment connecting both shoulders and the x-axis direction, and that is on a plane perpendicular to the y-axis. Further, the calculation unit 12 calculates an angle serving as an ability index of a right rotation motion or a left rotation motion of the neck, on the basis of the direction information on the face which is acquired by the data acquisition unit 11 and the calculated opening angle of the shoulder. This calculation processing will be described in more detail with reference to FIG. 18.

FIG. 18 is a diagram illustrating a method of calculating an ability index of the neck.

As an opening angle of the shoulder, the calculation unit 12 calculates an angle A11 that is formed by a line segment L11 connecting a left shoulder P11 and a right shoulder P12 and the positive direction of the x-axis, and that is on a plane (xz plane, the paper of FIG. 18) perpendicular to the y-axis. As an ability index of a right rotation motion of the neck, the calculation unit 12 calculates an angle (A11+A12) obtained by adding an angle A12, which is indicated by the direction information on the face which is acquired by the data acquisition unit 11, and an opening angle A11 between the shoulders. The calculated angle (A11+A12) becomes equal to an angle from a front direction L12 to a direction L13, which is obtained by a rightward rotation motion of the next from the front direction L12 by the test subject.

In addition, as an opening angle of the shoulder, the calculation unit 12 calculates an angle A13 that is formed by the line segment L11 connecting the left shoulder P11 and the right shoulder P12 and the negative direction of the x-axis, and that is on a plane (xz plane, the paper of FIG. 18) perpendicular to the y-axis. As an ability index of a left rotation motion of the neck, the calculation unit 12 calculates an angle (A13+A14) obtained by adding an angle A14, which is indicated by the direction information on the face which is acquired by the data acquisition unit 11, and an opening angle of the shoulder A13. The calculated angle (A13+A14) becomes equal to an angle from a front direction L14 to a direction L15, which is obtained by a left rotation motion of the next from the front direction L14 by the test subject.

A reporting unit 17 reports a determination result regarding whether or not the opening angle of the shoulder which is calculated by the calculation unit 12 is included in a predetermined angle range. The reporting unit 17 outputs a predetermined voice or a predetermined sound from a speaker connected to an input and output I/F 3 in a case where the opening angle of the shoulder is included in the predetermined angle range. The reporting unit 17 may output a predetermined sound or a predetermined voice in a case where the opening angle of the shoulder is not included in the predetermined angle range. However, a reporting method performed by the reporting unit 17 is not limited to the output of a voice. The reporting unit 17 may perform reporting by the output of a display.

A determination unit 13 determines whether or not the opening angle of the shoulder which is calculated by the calculation unit 12 is included in the predetermined angle range, and determines whether or not the test subject is positioned within the predetermined distance range from the three-dimensional sensor 7. The predetermined angle range is determined in advance from a restriction of the acquisition of the direction of the face by the data acquisition unit 11 and an average angle of the rotation motions of the neck. For example, the predetermined angle range is set to a range from equal to or greater than 28 degrees and equal to or less than 32 degrees. The predetermined distance range is determined in advance from the accuracy of acquisition of the direction of the face by the data acquisition unit 11, and the like. For example, the predetermined distance range is set to a range from 1.5 meters and 2.0 meters. The determination unit 13 can acquire a depth (distance from the three-dimensional sensor 7) of the test subject in a world coordinate system from a depth image.

[Example of Output]

Next, a concrete example of the output of a display by an output processing unit 14 according to the fourth example embodiment will be described.

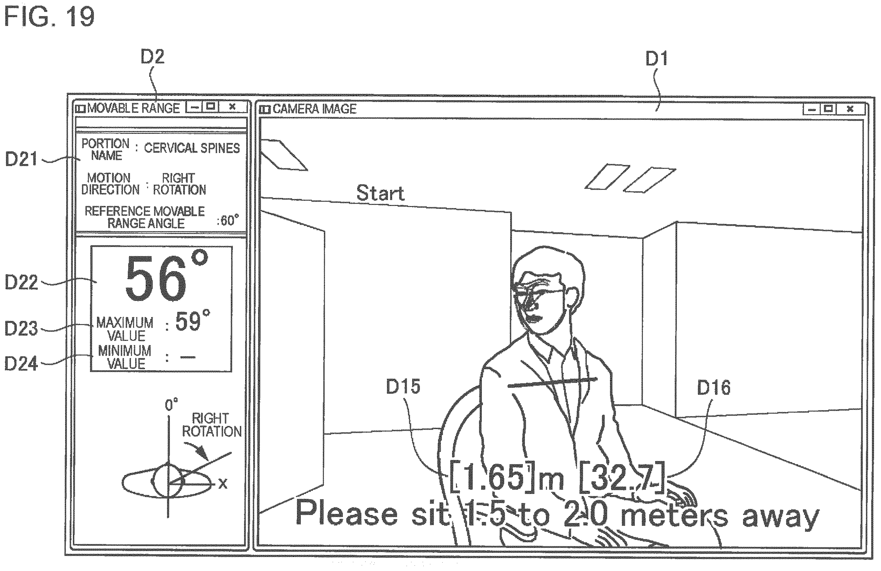

FIG. 19 is a diagram illustrating an example of the output of a display when a neck movable range is measured. The arrangement of display area of a video area D1, an explanation area D2, and the like is the same as those in FIG 4 and the like. The output processing unit 14 displays the fact that the right rotation motion of the neck is selected by the user in the area D21, displays angles calculated by the calculation unit 12 in an area D22, and displays a maximum value of the angles in an area D23. Meanwhile, also in FIG. 19, a minimum value may be displayed, similar to the examples in FIG. 4 and the like.

The output processing unit 14 displays a line segment connecting both shoulders so as to superimpose the line segment on a two-dimensional image while displaying the two-dimensional image in the video area D1 at any time, similar to FIG. 4 and the like. In addition, the output processing unit 14 displays a line segment connecting portions of the face on the basis of positional information on the portions of the face, which is obtained together with direction information on the face. Further, the output processing unit 14 displays the depth of the test subject which is acquired by the determination unit 13 in an area D15 (1.65 meters), and displays the opening angle of the shoulder which is calculated by the calculation unit 12 in an area D16 (32.7 degrees).

[Example of Operation/Measurement Method]

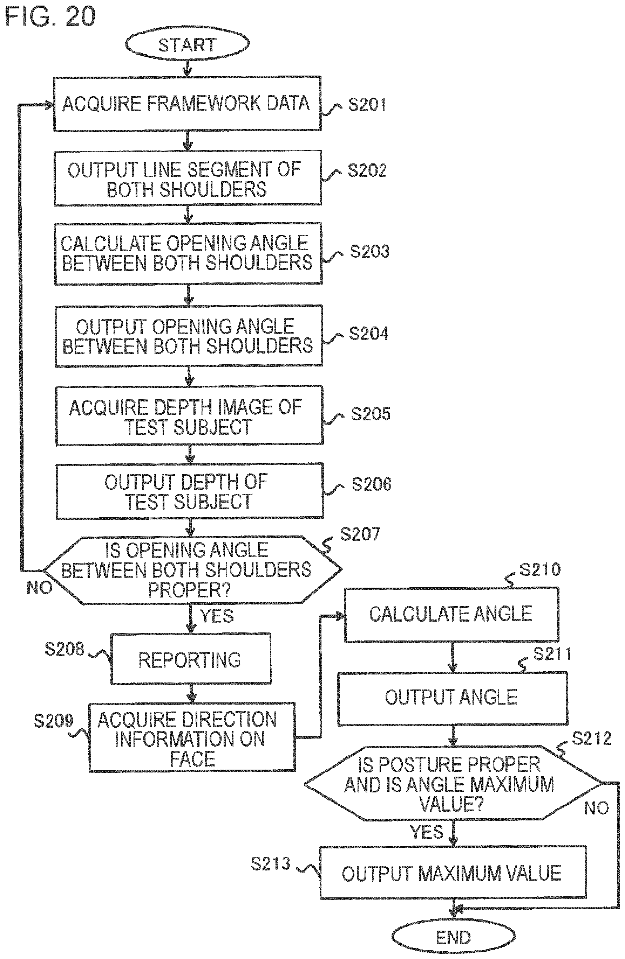

Hereinafter, the measurement method according to the fourth example embodiment will be described with reference to FIG. 20.

FIG. 20 is a flow chart illustrating an example of the operation of the measurement device 10 according to the fourth example embodiment. As illustrated in FIG. 20, the measurement method according to the fourth example embodiment is executed by at least one computer such as the measurement device 10. Processing steps illustrated in FIG 20 are the same as the processing contents of the above-described processing modules included in the measurement device 10, and thus details of the processing steps will be appropriately omitted.

The measurement device 10 determines any one of a right turning motion or a left turning motion which is selected by the user for the measurement of a neck movable range in executing the processing steps illustrated in FIG. 20. The measurement device 10 may sequentially execute the right turning motion and the left turning motion by automatically switching the motions.

The measurement device 10 acquires pieces of framework data of the test subject which is present within a visual field of the three-dimensional sensor 7 on the basis of information obtained from the three-dimensional sensor 7 (S201). The acquired pieces of framework data are pieces of positional information on a plurality of predetermined portions of limbs of the test subject which is used in (S202), and include positional information on both shoulders. The measurement device 10 sequentially acquires the pieces of framework data at predetermined cycles. A method of acquiring the framework data is as described above (data acquisition unit 11).

The measurement device 10 superimposes a line segment connecting both shoulders on two-dimensional images acquired from the three-dimensional sensor 7 on the basis of the framework data acquired in (S201) while sequentially displaying the two-dimensional images (S202). At this time, the measurement device 10 can use a correspondence relation between a world coordinate system indicated by the framework data and an image coordinate system of the two-dimensional image.

The measurement device 10 calculates an angle formed by the line segment connecting both shoulders and the x-axis direction on a plane perpendicular to the y-axis, as an opening angle of the shoulder on the basis of the positional information on both shoulders which is indicated by the framework data acquired in (S201) (S203). The measurement device 10 outputs the opening angle of the shoulder which is calculated in (S203) (S204). In the example in FIG. 19, the measurement device 10 displays the opening angle of the shoulder which is calculated in (S203) in an area D16.

The measurement device 10 acquires depth information on the test subject (S205). The acquired depth information indicates a distance in the world coordinate system from the three-dimensional sensor 7 to the test subject. The measurement device 10 outputs a depth of the test subject which is indicated by the depth information acquired in (S205) (S206). In the example in FIG. 19, the measurement device 10 displays the depth of the test subject in the area D15.

Subsequently, the measurement device 10 determines whether or not the opening angle of the shoulder which is calculated in (S203) is proper (S207). Specifically, the measurement device 10 determines whether or not the opening angle of the shoulder is included in a predetermined angle range. The predetermined angle range is as described above. In a case where the opening angle of the shoulder is not included in the predetermined angle range, that is, the opening angle of the shoulder is not proper (S207; NO), the measurement device 10 executes (S201) and the subsequent steps again.

In a case where the opening angle of the shoulder is included in the predetermined angle range (S207; YES), the measurement device 10 reports that the opening angle of the shoulder is proper (S208). A reporting method in (S208) is not limited. For example, the measurement device 10 performs reporting by a predetermined voice. In addition, in this example of operation, it is reported that the opening angle of the shoulder is proper, but it may be reported that the opening angle of the shoulder is not proper.

The measurement device 10 acquires the direction information on the face of the test subject on the basis of the information obtained from the three-dimensional sensor 7 (S209). A method of acquiring the direction information on the face is as described above. For example, when the recognition of the face is successful, the measurement device 10 outputs "Start" meaning the start of measurement as illustrated in FIG. 19. A signal for the start of measurement may be presented by a display, or may be presented by a voice or the like. In addition, the measurement device 10 may output "Ready" indicating a preparation stage until succeeding in the recognition of the face.

The measurement device 10 adds the opening angle of the shoulder which is calculated in (S203) and the angle indicated by the direction information on the face which is acquired in (S209) to calculate an angle serving as an ability index of a right rotation motion or a left rotation motion of the neck (S210). The measurement device 10 outputs the angle calculated in (S210) (S211). In the example in FIG. 19, the measurement device 10 displays the angle which is calculated in (S210) in the area D22. At this time, the measurement device 10 may output the angle in a state corresponding to a determination result regarding whether or not the depth of the test subject which is indicated by the depth information acquired in (S205) is proper. For example, the measurement device 10 displays the angle with a black character in a case where the depth of the test subject is within the predetermined distance range, and displays the angle with a red character in a case where the depth of the test subject is not within the predetermined distance range.

The measurement device 10 determines whether or not the depth of the test subject which is indicated by the depth information acquired in (S205) is proper and the angle calculated in (S210) indicates a maximum value (S212). The measurement device 10 determines whether or not the angle calculated in (S210) indicates the largest angle, among angles sequentially calculated, on the basis of the pieces of framework data sequentially acquired and the direction information on the face. In addition, the measurement device 10 determines whether or not the depth (distance) of the test subject is included within the predetermined distance range. The measurement device 10 determines that the depth of the test subject is proper in a case where the depth of the test subject is within the predetermined distance range, and determines that the depth of the test subject is not proper in other cases. The predetermined distance range is as described above.

In a case where it is determined that the depth of the test subject is proper and the calculated angle indicates a maximum value (S212; YES), the measurement device 10 outputs the angle calculated in (S210) as a maximum value (S213). For example, the measurement device 10 displays the angle calculated in (S210) in the area D23, as illustrated in the example in FIG. 19. On the other hand, in a case where it is determined that the depth of the test subject is not proper or the calculated angle does not indicate a maximum value (S212; NO), the measurement device 10 does not execute (S213). Further, in a case where the depth of the test subject is not proper, the measurement device 10 excludes the angle calculated in (S210) from the subsequent selection candidates of a maximum value. This is because there is a possibility that the accuracy of the acquired direction of the face of the test subject is low in a case where the depth of the test subject is not proper.

After it is determined that the opening angle of the shoulder is proper (S207; YES), the measurement device 10 can sequentially execute (S209) and the subsequent steps whenever a frame of a two-dimensional image and a frame of a depth image (distance image) from the three-dimensional sensor 7 are acquired. The processing steps of (S209) and the subsequent steps may be executed at intervals longer than a cycle for acquiring the frames. The order of execution of the processing steps in the measurement method of this example embodiment is not limited to the example illustrated in FIG. 20. The order of execution of the processing steps can be changed within a range as long as the change does not affect the content. For example, (S202) may be executed after (S203). In addition, (S205) and (S206) may be executed prior to (S202).

[Advantageous Effects of Fourth EXAMPLE EMBODIMENT]

In the fourth example embodiment, opening angles of both shoulders is calculated on the basis of positional information on both shoulders which is indicated by framework data. The opening angles of both shoulders indicates a degree at which the right shoulder or the left shoulder is pulled in a direction (z-axis direction) receding from the three-dimensional sensor 7 from a state where the test subject faces the three-dimensional sensor 7. The direction (angle) of the face of the test subject is determined on the basis of information from the three-dimensional sensor 7, and an angle serving as an ability index of a right rotation motion or a left rotation motion of the neck is calculated by addition of the determined direction of the face and the opening angles of both shoulders. Thereby, there is a limit to the direction (angle) of the face which is capable of being determined on the basis of the information from the three-dimensional sensor 7. Thus, according to the fourth example embodiment, it is possible to accurately measure an ability index of a right rotation motion or a left rotation motion of the neck by combining with the opening angles of both shoulders.

Further, in the fourth example embodiment, it is determined whether or not the opening angle of the shoulder is within a predetermined angle range or the test subject is positioned within the predetermined distance range from the three-dimensional sensor 7. A determination result regarding whether or not the opening angle of the shoulder is included within the predetermined angle range is reported, and an angle is output in a state corresponding to a determination result regarding whether or not the test subject is positioned within the predetermined distance range from the three-dimensional sensor 7. Thereby, the test subject confirms the reporting and the output, and thus it is possible to measure a movable range of the neck in a proper posture. That is, according to the fourth example embodiment, it is possible to improve usability of the measurement of the movable range of the neck.

[Supplementary Example]

In the examples of output illustrated in FIG. 4 and the like, the three-dimensional sensor 7 is disposed so as to face a horizontal direction in the real world. However, the three-dimensional sensor 7 may be disposed so as to face a vertical direction for the measurement of a hip movable range or the like. In this case, in positional information of a world coordinate system obtained from the three-dimensional sensor 7, the vertical direction in the real world is set to be a z-axis, and a horizontal plane in the real world is set to be an xy plane.

For example, the operation detection unit 15 detects a user operation for selecting a flexion motion for the measurement of a hip movable range. An ability index of a flexion motion of a hip is measured for each of the left foot and the right foot. The operation detection unit 15 may detect an operation of selecting which one of the left and the right is measured, with respect to the flexion motion of the hip. In this case, the data acquisition unit 11 acquires direction information on the waist and the knee of the test subject on the basis of the positional information obtained from the three-dimensional sensor. As an ability index of the flexion motion of the hip, the calculation unit 12 calculates an angle that is on a plane (yz plane) perpendicular to the x-axis direction and that is formed by a line segment in the negative direction of the y-axis with the position of the waist as an endpoint and a line segment connecting the position of the waist and the position of the knee, on the basis of the positional information acquired by the data acquisition unit 11.

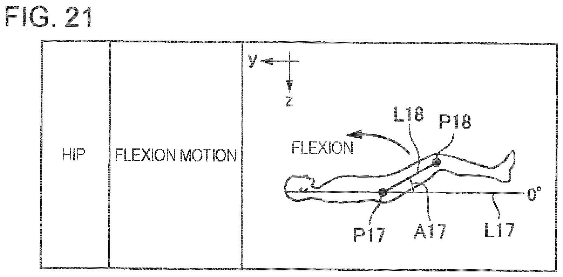

FIG. 21 is a diagram illustrating a method of calculating an ability index of the hip. The calculation unit 12 calculates an angle A17 that is on a plane (yz plane, the paper of FIG. 21) perpendicular to the x-axis and that is formed by a line segment L17 in the negative direction of the y-axis with the position P17 of the right waist as an endpoint and a line segment L18 connecting a position P17 of the right waist and a position P18 of the right knee, as an ability index of a flexion motion of the right hip. The calculation unit 12 similarly calculates an ability index of a flexion motion of the left hip.

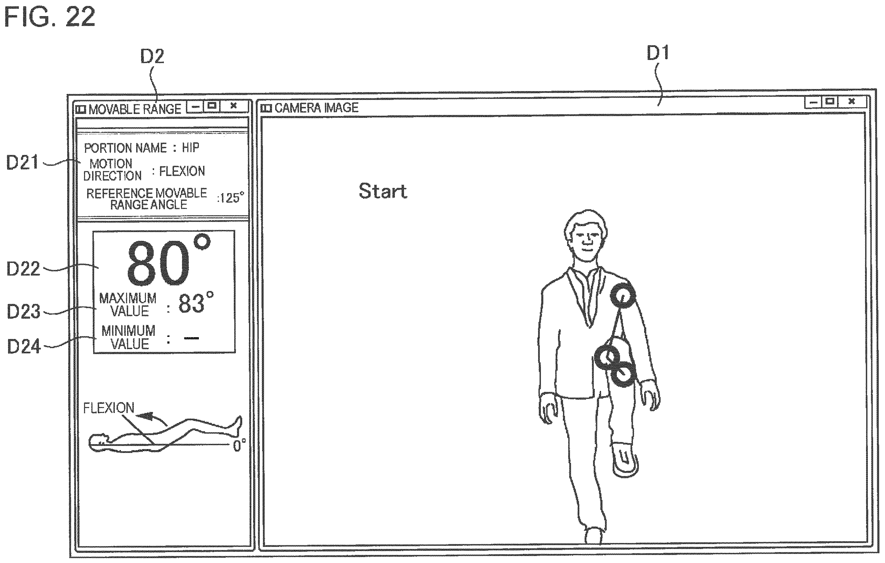

FIG. 22 is a diagram illustrating an example of the output of a display by the output processing unit 14 according to a supplementary example.

In this case, the output processing unit 14 displays the flexion motion of the hip being selected by the user in the area D21, displays angles calculated by the calculation unit 12 in the area D22, and displays a maximum value of the angles in the area D23. The output processing unit 14 superimposes a mark on the positions of the left knee, the left waist, and the left shoulder on a two-dimensional image while displaying the two-dimensional image in the video area D1 at any time, similar to FIG. 4 and the like. In the example in FIG. 22, the test subject is measured in a state of lying down on the floor.

In the above description, a description is given of an example in which the three-dimensional sensor 7 is realized as a sensor in which a visible light camera and a depth sensor are integrated, and the measurement device 10 (data acquisition unit 11) acquires a two-dimensional image and a depth image from the three-dimensional sensor 7. However, a method of realizing the three-dimensional sensor 7 is not limited, and the three-dimensional sensor 7 may be realized as a plurality of imaging devices that image a marker attached to the test subject and may include a plurality of infrared sensors that detect the position of an infrared marker attached to the test subject. In this case, a marker is attached to necessary portions of limbs of the test subject before measurement. The measurement device 10 (data acquisition unit 11) can detect the position of the marker by the plurality of imaging devices or infrared sensors to acquire positional information (framework data) on a plurality of predetermined portions of the limbs of the test subject. Further, a distance (interval) between markers in the real world is measured in advance when attaching a marker to each portion, and thus it is possible to calculate a distance between portions in a world coordinate system and a depth to the test subject from the acquired positional information. However, in a case where a depth sensor is not included, a depth image is not acquired, and thus positional information acquired by the detection of a marker may be used.

[Fifth EXAMPLE EMBODIMENT]

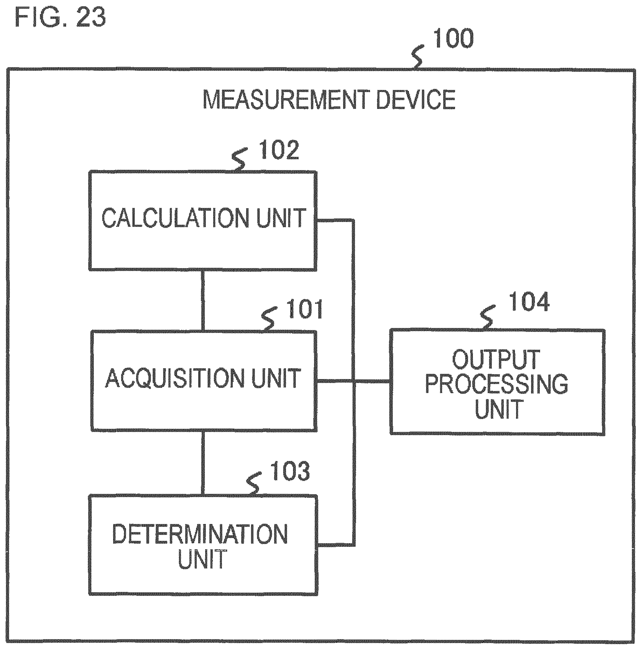

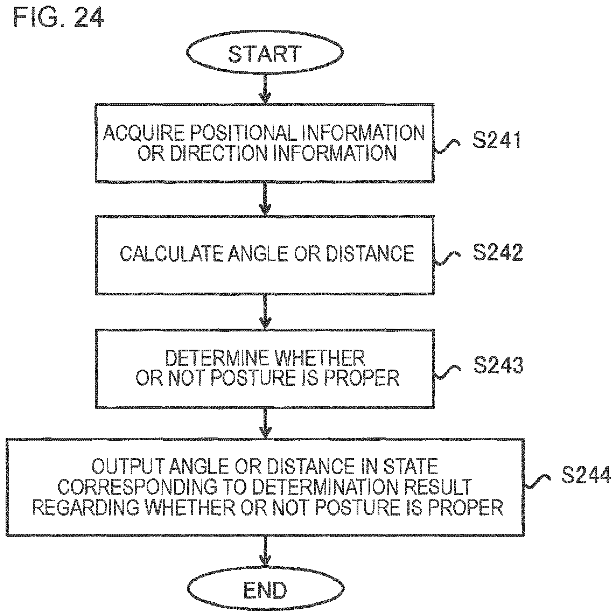

Hereinafter, a measurement device and a measurement method according to a fifth example embodiment will be described with reference to FIGS. 23 and 24. In addition, the fifth example embodiment may relate to a program causing at least one computer to execute the measurement method, and may relate to a storage medium readable by a computer having the program recorded thereon.

FIG. 23 is a schematic diagram illustrating an example of a processing configuration of a measurement device 100 according to the fifth example embodiment. As illustrated in FIG. 23, the measurement device 100 includes an acquisition unit 101, a calculation unit 102, a determination unit 103, and an output processing unit 104. The measurement device 100 has the same hardware configuration as that of, for example, the above-described measurement device 10 illustrated in FIG. 1, and the above-described processing modules are realized by the program being processed similar to the measurement device 10.

The acquisition unit 101 acquires positional information or direction information on a predetermined portion in a limb of a test subject. For example, the acquisition unit 101 acquires the positional information or the direction information on the basis of information obtained from a three-dimensional sensor 7. In addition, the acquisition unit 101 may acquire the positional information or the direction information from another computer. A specific example of the acquisition unit 101 is the above-described data acquisition unit 11.

The calculation unit 102 calculates an angle or a distance serving as an ability index of the limb of the test subject on the basis of the positional information or the direction information acquired by the acquisition unit 101. A specific example of the calculation unit 102 is the above-described calculation unit 12, and the calculation unit 102 calculates the angle or the distance by any one or more methods of the above-described methods or another method. The ability index of the limb of the test subject which is calculated by the calculation unit 102 may be any one or more ability indexes of a flexion motion, an extension motion, an abduction motion, an adduction motion, an external rotation motion, an internal rotation motion, a horizontal extension motion, and a horizontal flexion motion of a shoulder, a flexion motion of a hip, a right rotation motion and a left rotation motion of a neck, a flexion motion of a thoracic and lumbar spine, and a functional reach test, and may be an ability index of another portion determined in Non-Patent Document 1.

The determination unit 103 determines whether or not the posture of the test subject is proper, on the basis of the positional information or the direction information acquired by the acquisition unit 101. A specific example of the determination unit 103 is the above-described determination unit 13, and the determination unit 103 determines whether or not the posture of the test subject is proper by any one or more methods of the above-described methods or another method. A correct posture of the test subject is determined in advance in accordance with a portion of a limb to be measured and a motion type.

The output processing unit 104 outputs the angle or the distance which is calculated by the calculation unit 102 in a state corresponding to a determination result regarding whether or not the posture of the test subject is proper. An output method of the output processing unit 104 is not limited. The output processing unit 104 may display the angle or the distance on a display device 5, may cause a printing device connected to the communication unit 4 to print the angle or the distance, or may transmit the angle or the distance to another computer through the communication unit 4. In addition, the output processing unit 104 can also output a sound for reading the angle or the distance.

A state where the angle or the distance is output may be any state as long as a determination result regarding the propriety of the posture of the test subject is reflected thereon. The output processing unit 104 can switch a color, a font type, the thickness of a line, a background, or a position which is used for the output of the angle or the distance in accordance with the determination result regarding whether or not the posture is proper. Further, the output processing unit 104 may output a sound for reading the angle or the distance by a different sound volume.

FIG. 24 is a flow chart illustrating an example of the operation of the measurement device 100 according to the fifth example embodiment. As illustrated in FIG. 24, the measurement method according to the fifth example embodiment is executed by at least one computer such as the measurement device 100. Processing steps illustrated in FIG. 24 are the same as the processing contents of the above-described processing modules included in the measurement device 100, and thus details of the processing steps will be appropriately omitted.

The measurement method according to this example embodiment includes processing steps (S241), (S242), (S243), and (S244) as illustrated in FIG. 24.

In (S241), the measurement device 100 acquires positional information or direction information on a predetermined portion in a limb of a test subject.

In (S242), the measurement device 100 calculates an angle or a distance serving as an ability index of the limb of the test subject on the basis of the positional information or the direction information which is acquired in (S241).

In (S243), the measurement device 100 determines whether or not the posture of the test subject is proper, on the basis of the positional information or the direction information which is acquired in (S241).

In (S244), the measurement device 100 outputs the angle or the distance which is calculated in (S242) in a state corresponding to a result of the determination (S243) regarding whether or not the posture of the test subject is proper.

In the fifth example embodiment, the angle or the distance serving as the ability index of the limb of the test subject is calculated on the basis of the positional information or the direction information on the predetermined portion in the limb of the test subject, and it is determined whether or not the posture of the test subject is proper. The calculated angle or distance is output in a state corresponding to the determination result regarding whether or not the posture of the test subject is proper. Therefore, according to the fifth example embodiment, a person viewing the output can be caused to immediately recognize whether the test subject performs measurement in a proper posture, and thus it is possible to prompt the test subject to perform measurement in a correct posture. As a result, according to the fifth example embodiment, it is possible to accurately measure a limb ability of the test subject.

Meanwhile, in the plurality of flow charts used in the above description, a plurality of steps (processes) are described in order, but the order of execution of the steps executed in this example embodiment is not limited to the described order. In this example embodiment, the order of the steps illustrated in the drawing can be changed within a range as long as the change does not affect the content. In addition, the above-described example embodiments and supplementary example can be combined with each other in a range in which the contents thereof do not conflict with each other, and the above-described example embodiments and supplementary example may be formed by only respective individual configurations.

## Claims

1. A measurement device (10) comprising:

    an acquisition unit (11) adapted to acquire positional information or direction information on a predetermined portion in a limb of a test subject;
    a calculation unit (12) adapted to calculate angles or distances serving as ability indexes of the limb of the test subject on the basis of the acquired positional information or direction information;
    a determination unit (13) adapted to determine whether or not a posture of the test subject is proper, on the basis of the acquired positional information or direction information; and
    an output processing unit (14) adapted to output the calculated angles or distances in a state corresponding to a determination result regarding whether or not the posture of the test subject is proper;
    **characterized in that** the acquisition unit (11) is adapted to acquire positional information on a floor and positional information on a toe of the test subject on the basis of a depth image of a lower half of the test subject which is obtained from a three-dimensional sensor, and is adapted to acquire positional information on a fingertip of the hand of the test subject on the basis of a depth image of the test subject, in an image region having an x coordinate larger than the x-coordinate of the position of the toe in the depth image, wherein the toe indicates the positive direction of the x-axis of a coordinate system of the depth image ,
    wherein the calculation unit (12) is adapted to calculate a distance from the fingertip of the hand and the floor on the basis of the positional information on the floor and the positional information on the fingertip of the hand of the test subject,
    wherein the measurement device further comprises an image acquisition unit adapted to successively acquire images including the test subject from the three-dimensional sensor,
    wherein the output processing unit (14) is adapted to superimpose a mark on the position of the toe of the test subject on the acquired images to be displayed on the basis of the acquired positional information on the toe while sequentially displaying the acquired images, fixedly superimposing the mark on the sequentially displayed images by detection of a predetermined event after the superimposition of the mark, and performing coloring based on the depth information regarding the test subject on an image region having an x-coordinate larger than the x coordinate of the position of the toe in the depth image included in the sequentially displayed images.

2. The measurement device (10) according to claim 1, wherein the output processing unit (14) is further adapted to output a display in which a line segment is superimposed on an image including the test subject in a state corresponding to the determination result regarding whether or not the posture of the test subject is proper, the line segment connecting a plurality of predetermined portions respectively corresponding to a plurality of pieces of positional information used for the calculation of the angles or the distances.

3. The measurement device (10) according to claim 1 or 2,

wherein the acquisition unit (11) is adapted to sequentially acquire the positional information or the direction information on the predetermined portion in the limb of the test subject,
wherein the calculation unit (12) is adapted to sequentially calculate the angles or the distances on the basis of the sequentially acquired positional information or direction information, and
wherein the output processing unit (14) is adapted to exclude an angle or distance calculated when it is determined that the posture of the test subject is improper from selection candidates of a maximum value or a minimum value among the angles or the distances sequentially calculated with respect to the test subject while outputting the maximum value or the minimum value.

4. The measurement device (10) according to any one of claims 1 to 3,

wherein the acquisition unit (11) is adapted to acquire pieces of positional information on a shoulder and a hand of the test subject on the basis of information obtained from a three-dimensional sensor, and
wherein the calculation unit (12) is adapted to calculate as an ability index of a flexion motion, an extension motion, an abduction motion, or an adduction motion of the shoulder on the basis of the acquired positional information, the angle being formed by a vertically downward line segment of a visual field of the three-dimensional sensor with the position of the shoulder as an endpoint and a line segment connecting a position of the shoulder and a position of the hand, and the angle being on a plane perpendicular to a horizontal direction or a depth direction of the visual field of the three-dimensional sensor.

5. The measurement device (10) according to any one of claims 1 to 4,

wherein the acquisition unit (11) is adapted to acquire pieces of positional information on an elbow and the hand of the test subject on the basis of the information obtained from the three-dimensional sensor, and
wherein the calculation unit (12) is adapted to calculate an angle as an ability index of an external rotation motion or an internal rotation motion of the shoulder on the basis of the acquired positional information, the angle being formed by a line segment in a direction opposite to the depth direction of the visual field of the three-dimensional sensor with the position of the elbow as an endpoint and a line segment connecting a position of the elbow and the position of the hand, the angle being on a plane perpendicular to a vertical direction of the visual field of the three-dimensional sensor.

6. The measurement device (10) according to any one of claims 1 to 5,

wherein the acquisition unit (11) is adapted to acquire pieces of positional information on the shoulder and the hand of the test subject on the basis of the information obtained from the three-dimensional sensor, and
wherein the calculation unit (12) is adapted to calculate an angle as an ability index of a horizontal extension motion or a horizontal flexion motion of the shoulder on the basis of the acquired positional information, the angle being formed by a line segment in a horizontal direction of the visual field of the three-dimensional sensor with the position of the shoulder as an endpoint and a line segment connecting the position of the shoulder and the position of the hand, the angle being on a plane perpendicular to the vertical direction of the visual field of the three-dimensional sensor.

7. The measurement device (10) according to any one of claims 1 to 6,

wherein the acquisition unit is adapted to acquire pieces of positional information on a waist and a knee of the test subject on the basis of the information obtained from the three-dimensional sensor, and
wherein the calculation unit (12) is adapted to calculate an angle as an ability index of a flexion motion of a hip on the basis of the acquired positional information, the angle being formed by a vertically downward line segment of the visual field of the three-dimensional sensor with the position of the waist as an endpoint and a line segment connecting a position of the waist and a position of the knee, the angle being on a plane perpendicular to the horizontal direction of the visual field of the three-dimensional sensor.

8. The measurement device (10) according to any one of claims 1 to 7,

wherein the acquisition unit (11) is adapted to acquire direction information on a face and positional information on both shoulders of the test subject on the basis of the information obtained from the three-dimensional sensor, and

wherein the calculation unit (12) is adapted to calculate an angle, which is formed by a line segment connecting both the shoulders and the horizontal direction of the visual field of the three-dimensional sensor and is on a plane perpendicular to the vertical direction of the visual field of the three-dimensional sensor, as an opening angle of the shoulder on the basis of the acquired positional information, and to calculate an angle serving as an ability index of a right rotation motion or a left rotation motion of a neck on the basis of the acquired direction information and the calculated opening angle of the shoulder.

9. The measurement device (10) according to claim 8, further comprising:
a reporting unit adapted to report a determination result regarding whether or not the calculated opening angle of the shoulder is included in a predetermined angle range.

10. The measurement device (10) according to claim 8 or 9, further comprising:
wherein the determination unit (13) is adapted to determine whether or not the calculated opening angle of the shoulder is included in a predetermined angle range, and whether or not the test subject is positioned within a predetermined distance range from the three-dimensional sensor.

11. The measurement device (10) according to any one of claims 1 to 10,

wherein the acquisition unit (11) is adapted to sequentially acquire depth images of a whole body of the test subject on the basis of the information obtained from the three-dimensional sensor, sequentially acquire pieces of positional information on the fingertip of the hand of the test subject on the basis of the depth images, and hold positional information on the fingertip of the hand which is acquired during the detection of the predetermined event, and

wherein the calculation unit (12) is adapted to calculate a distance in the horizontal direction of the visual field of the three-dimensional sensor between the fingertips of the hand, on the basis of the held positional information on the fingertip of the hand and newly acquired positional information on the fingertip of the hand.

12. A measurement method executed by at least one computer, the measurement method comprising:

acquiring (S241) positional information or direction information on a predetermined portion in a limb of a test subject;
calculating (S242) angles or distances serving as ability indexes of the limb of the test subject on the basis of the acquired positional information or direction information;
determining (S243) whether or not a posture of the test subject is proper, on the basis of the acquired positional information or direction information; and
outputting (S244) the calculated angles or distances in a state corresponding to a determination result regarding whether or not the posture of the test subject is proper;
**characterized in that** acquiring (S241) positional information or direction information comprises acquiring positional information on a floor and positional information on a toe of the test subject on the basis of a depth image of a lower half of the test subject which is obtained from a three-dimensional sensor, and acquiring positional information on a fingertip of the hand of the test subject on the basis of a depth image of the test subject, in an image region having an x-coordinate larger than the x coordinate of the position of the toe in the depth image, wherein the toe indicates the positive direction of the x-axis of a coordinate system of the depth image,
wherein calculating (S242) angles or distances comprises calculating a distance from the fingertip of the hand and the floor on the basis of the positional information on the floor and the positional information on the fingertip of the hand of the test subject,
successively acquiring images including the test subject from the three-dimensional sensor,

wherein outputting (S244) the calculated angles or distances comprises superimposing a mark on the position of the toe of the test subject on the acquired images to be displayed on the basis of the acquired positional information on the toe while sequentially displaying the acquired images, fixedly superimposing the mark on the sequentially displayed images by detection of a predetermined event after the superimposition of the mark, and performing coloring based on the depth information regarding the test subject on an image region having an x-coordinate larger than the x coordinate of the position of the toe in the depth image included in the sequentially displayed images.

**13.** A program causing at least one computer to execute the measurement method according to claim 12.

**Patentansprüche**

**1.** Messvorrichtung (10), umfassend:

eine Erfassungseinheit (11), die dazu ausgelegt ist, Positionsinformation oder Richtungsinformation über einen vorbestimmten Bereich in einer Extremität eines Testsubjekts zu erfassen;
eine Recheneinheit (12), die dazu ausgelegt ist, auf der Basis der erfassten Positionsinformation oder Richtungsinformation Winkel oder Distanzen zu berechnen, die als Befähigungsindizes der Extremität des Testsubjekts dienen;
eine Bestimmungseinheit (13), die dazu ausgelegt ist, auf der Basis der erfassten Positionsinformation oder Richtungsinformation zu bestimmen, ob oder ob nicht eine Haltung des Testsubjekts richtig ist; und
eine Ausgabeverarbeitungseinheit (14), die dazu ausgelegt ist, die berechneten Winkel oder Distanzen in einem Zustand entsprechend einem Bestimmungsergebnis dahingehend auszugeben, ob oder ob nicht die Haltung des Testsubjekts richtig ist;
**dadurch gekennzeichnet, dass** die Erfassungseinheit (11) dazu ausgelegt ist, Positionsinformation über einen Boden und Positionsinformation über einen Zeh des Testsubjekts auf der Basis eines Tiefenbilds einer unteren Hälfte des Testsubjekts zu erfassen, das von einem dreidimensionalen Sensor erhalten ist, und dazu ausgelegt ist, Positionsinformation über eine Fingerspitze der Hand des Testsubjekts auf der Basis eines Tiefenbilds des Testsubjekts in einer Bildregion zu erfassen, die eine x-Koordinate größer als die x-Koordinate der Position des Zehs in dem Tiefenbild hat, wobei der Zeh die positive Richtung der x-Achse eines Koordinatensystems des Tiefenbilds angibt,
wobei die Recheneinheit (12) dazu ausgelegt ist, auf der Basis der Positionsinformation des Bodens und der Positionsinformation der Fingerspitze der Hand des Testsubjekts eine Distanz von der Fingerspitze der Hand und dem Boden zu berechnen,
wobei die Messvorrichtung ferner eine Bilderfassungseinheit umfasst, die dazu ausgelegt ist, sukzessive von dem dreidimensionalen Sensor Bilder zu erfassen, die das Testsubjekt enthalten,
wobei die Ausgabeverarbeitungseinheit (14) dazu ausgelegt ist, eine Markierung zu überlagern an der Position des Zehs des Testsubjekts auf den erfassten Bildern zur Anzeige auf der Basis der erfassten Positionsinformation über den Zeh, während die erfassten Bilder sukzessive angezeigt werden, wobei die Markierung fest auf den sequentiell angezeigten Bildern überlagert wird durch Detektion eines vorbestimmten Ereignisses nach der Überlagerung der Markierung, und Durchführen einer Einfärbung basierend auf der Tiefeninformation im Hinblick auf das Testsubjekt auf einer Bildregion mit einer x-Koordinate größer als die x-Koordinate der Position des Zehs in dem Tiefenbild, das in den sequentiell angezeigten Bildern enthalten ist.

**2.** Messvorrichtung (10) nach Anspruch 1, wobei die Ausgabeverarbeitungseinheit (14) ferner dazu ausgelegt ist, eine Anzeige auszugeben, in der ein Liniensegment auf einem Bild überlagert ist, das das Testsubjekt enthält, in einem Zustand entsprechend dem Bestimmungsergebnis dahingehend, ob oder ob nicht die Haltung des Testsubjekts richtig ist, wobei das Liniensegment eine Mehrzahl von vorbestimmten Bereichen verbindet, die jeweils einer Mehrzahl von Teilen von Positionsinformation entsprechen, die für die Berechnung der Winkel oder der Distanzen verwendet wird.

**3.** Messvorrichtung (10) nach Anspruch 1 oder 2,
wobei die Erfassungseinheit (11) dazu ausgelegt ist, sequentiell die Positionsinformation oder die Richtungsinformation über den vorbestimmten Bereich in der Extremität des Testsubjekts zu erfassen,
wobei die Recheneinheit (12) dazu ausgelegt ist, sequentiell die Winkel oder die Distanzen auf der Basis der sequentiell erfassten Positionsinformation oder Richtungsinformation zu berechnen, und
wobei die Ausgabeverarbeitungseinheit (14) dazu ausgelegt ist, einen berechneten Winkel oder Distanz auszuschließen, wenn bestimmt wird, dass die Haltung des Testsubjekts nicht richtig ist, und zwar von Auswahlkandidaten eines Maximalwerts oder eines Minimalwerts aus den Winkeln oder den Distanzen, die sequentiell berechnet werden im Hinblick auf das Testsubjekt während der Ausgabe des Maximalwerts oder des Minimalwerts.

4. Messvorrichtung (10) nach einem der Ansprüche 1 bis 3,
wobei die Erfassungseinheit (11) dazu ausgelegt ist, Teile von Positionsinformation über eine Schulter und eine Hand des Testsubjekts auf der Basis von Information zu erfassen, die von einem dreidimensionalen Sensor erhalten wird, und
wobei die Recheneinheit (12) dazu ausgelegt ist, einen Befähigungsindex einer Beugungsbewegung, einer Streckungsbewegung, einer Abduktionsbewegung oder einer Adduktionsbewegung der Schulter auf der Basis der erfassten Positionsinformation zu berechnen, wobei der Winkel gebildet ist durch ein Liniensegment eines Sichtfelds des dreidimensionalen Sensors vertikal abwärts mit der Position der Schulter als ein Endpunkt und ein Liniensegment, das eine Position der Schulter und eine Position der Hand verbindet, und der Winkel auf einer Ebene orthogonal zu einer horizontalen Richtung oder einer Tiefenrichtung des Sichtfelds des dreidimensionalen Sensors ist.

5. Messvorrichtung (10) nach einem der Ansprüche 1 bis 4,
wobei die Erfassungseinheit (11) dazu ausgelegt ist, Teile von Positionsinformation über einen Ellbogen und die Hand des Testsubjekts auf der Basis der Information zu erfassen, die von dem dreidimensionalen Sensor erhalten wird, und
wobei die Recheneinheit (12) dazu ausgelegt ist, einen Winkel als einen Befähigungsindex einer Außendrehbewegung oder einer Innendrehbewegung der Schulter zu berechnen auf der Basis der erfassten Positionsinformation, wobei der Winkel gebildet wird durch ein Liniensegment in einer Richtung entgegengesetzt zu der Tiefenrichtung des Sichtfelds des dreidimensionalen Sensors mit der Position des Ellbogens als ein Endpunkt und ein Liniensegment, das eine Position des Ellbogens und die Position der Hand verbindet, wobei der Winkel auf einer Ebene orthogonal zu einer vertikalen Richtung des Sichtfelds des dreidimensionalen Sensors ist.

6. Messvorrichtung (10) nach einem der Ansprüche 1 bis 5,
wobei die Erfassungseinheit (11) dazu ausgelegt ist, Teile von Positionsinformation über die Schulter und die Hand des Testsubjekts auf der Basis der Information zu erfassen, die von dem dreidimensionalen Sensor erhalten wird, und
wobei die Recheneinheit (12) dazu ausgelegt ist, einen Winkel als einen Befähigungsindex einer horizontalen Streckbewegung oder einer horizontalen Beugungsbewegung der Schulter zu berechnen auf der Basis der erfassten Positionsinformation, wobei der Winkel gebildet wird durch ein Liniensegment in einer horizontalen Richtung des Sichtfelds des dreidimensionalen Sensors mit der Position der Schulter als ein Endpunkt und ein Liniensegment, das die Position der Schulter und die Position der Hand verbindet, wobei der Winkel auf einer Ebene orthogonal zu der vertikalen Richtung des Sichtfelds des dreidimensionalen Sensors ist.

7. Messvorrichtung (10) nach einem der Ansprüche 1 bis 6,
wobei die Erfassungeinheit dazu ausgelegt ist, Teile von Positionsinformation über eine Hüfte und ein Knie des Testsubjekts auf der Basis der Information zu erfassen, die von dem dreidimensionalen Sensor erhalten wird, und
wobei die Recheneinheit (12) dazu ausgelegt ist, einen Winkel als einen Befähigungsindex einer Beugungsbewegung einer Hüfte zu berechnen auf der Basis der erfassten Positionsinformation, wobei der Winkel gebildet wird durch ein Liniensegment des Sichtfelds des dreidimensionalen Sensors vertikal abwärts mit der Position der Hüfte als ein Endpunkt und ein Liniensegment, das eine Position der Hüfte und eine Position des Knies verbindet, wobei der Winkel auf einer Ebene orthogonal zu der horizontalen Richtung des Sichtfelds des dreidimensionalen Sensors ist.

8. Messvorrichtung (10) nach einem der Ansprüche 1 bis 7,
wobei die Erfassungeinheit (11) dazu ausgelegt ist, Richtungsinformation über ein Gesicht und Positionsinformation über beide Schultern des Testsubjekts auf der Basis der Information zu erfassen, die von dem dreidimensionalen Sensor erhalten wird, und
wobei die Recheneinheit (12) dazu ausgelegt ist, einen Winkel zu berechnen, der gebildet wird durch ein Liniensegment, das die beiden Schultern und die horizontale Richtung des Sichtfelds des dreidimensionalen Sensors verbindet und auf einer Ebene orthogonal zu der vertikalen Richtung des Sichtfelds des dreidimensionalen Sensors ist, als ein Öffnungswinkel der Schulter auf der Basis der erfassten Positionsinformation, und einen Winkel zu berechnen, der als ein Befähigungsindex einer Rechtsdrehbewegung oder einer Linksdrehbewegung eines Halses dient, auf der Basis der erfassten Richtungsinformation und des berechneten Öffnungswinkel der Schulter.

9. Messvorrichtung (10) nach Anspruch 8, ferner umfassend:

eine Berichtseinheit, die dazu ausgelegt ist, ein Bestimmungsergebnis dahingehend zu berichten, ob oder ob nicht der berechnete Öffnungswinkel der Schulter in einem vorbestimmten Winkelbereich enthalten ist.

10. Messvorrichtung (10) nach Anspruch 8 oder 9, ferner umfassend:

   wobei die Bestimmungseinheit (13) dazu ausgelegt ist zu bestimmen, ob oder ob nicht der berechnete Öffnungswinkel der Schulter in einem vorbestimmten Winkelbereich enthalten ist, und ob oder ob nicht das Testsubjekt innerhalb eines vorbestimmten Distanzbereichs von dem dreidimensionalen Sensor positioniert ist.

11. Messvorrichtung (10) nach einem der Ansprüche 1 bis 10,

   wobei die Erfassungseinheit (11) dazu ausgelegt ist, sequentiell Tiefenbilder eines ganzen Körpers des Testsubjekts auf der Basis der Information zu erfassen, die von dem dreidimensionalen Sensor erhalten wird, sequentiell Teile von Positionsinformation über die Fingerspitze der Hand des Testsubjekts auf der Basis der Tiefenbilder zu erfassen, und Positionsinformation über die Fingerspitze der Hand zu halten, die während der Detektion des vorbestimmten Ereignisses erfasst wird, und

   wobei die Recheneinheit (12) dazu ausgelegt ist, eine Distanz in der horizontalen Richtung des Sichtfelds des dreidimensionalen Sensors zwischen den Fingerspitzen der Hand auf der Basis der gehaltenen Positionsinformation über die Fingerspitze der Hand und neu erfasste Positionsinformation über die Fingerspitze der Hand zu berechnen.

12. Messverfahren, das durch wenigstens einen Computer ausgeführt wird, wobei das Messverfahren umfasst:

   Erfassen (S241) von Positionsinformation oder Richtungsinformation über einen vorbestimmten Bereich in einer Extremität eines Testsubjekts;
   Berechnen (S242) von Winkeln oder Distanzen, die als Befähigungsindizes der Extremität des Testsubjekts dienen, auf der Basis der erfassten Positionsinformation oder Richtungsinformation;
   Bestimmen (S243), ob oder ob nicht eine Haltung des Testsubjekts richtig ist, auf der Basis der erfassten Positionsinformation oder Richtungsinformation; und
   Ausgeben (S244) der berechneten Winkel oder Distanzen in einem Zustand entsprechend einem Bestimmungsergebnis dahingehend, ob oder ob nicht die Haltung des Testsubjekts richtig ist;
   **dadurch gekennzeichnet, dass** das Erfassen (S241) von Positionsinformation oder Richtungsinformation ein Erfassen von Positionsinformation über einen Boden und Positionsinformation über einen Zeh des Testsubjekts auf der Basis eines Tiefenbilds einer unteren Hälfte des Testsubjekts umfasst, das von einem dreidimensionalen Sensor erhalten wird, und Erfassen von Positionsinformation über eine Fingerspitze der Hand des Testsubjekts auf der Basis einen Tiefenbilds des Testsubjekts in einem Bildbereich, der eine x-Koordinate größer als die x-Koordinate der Position des Zehs in dem Tiefenbild hat, wobei der Zeh die positive Richtung der x-Achse eines Koordinatensystems des Tiefenbilds angibt,
   wobei das Berechnen (S242) von Winkeln oder Distanzen ein Berechnen einer Distanz von der Fingerspitze der Hand und dem Boden auf der Basis der Positionsinformation über den Boden und die Positionsinformation über die Fingerspitze der Hand des Testsubjekts umfasst,
   sukzessives Erfassen von Bildern, die das Testsubjekt enthalten, von dem dreidimensionalen Sensor,
   wobei das Ausgeben (S244) der berechneten Winkel oder Distanzen ein Überlagern einer Markierung umfasst an der Position des Zehs des Testsubjekts auf den erfassten Bildern zur Anzeige auf der Basis der erfassten Positionsinformation über den Zeh, während die erfassten Bilder sequentiell angezeigt werden, ein festes Überlagern der Markierung auf den sequentiell angezeigten Bildern durch Detektion eines vorbestimmten Ereignisses nach der Überlagerung der Markierung, und ein Durchführen einer Einfärbung basierend auf der Tiefeninformation im Hinblick auf das Testsubjekt auf einer Bildregion mit einer x-Koordinate größer als die x-Koordinate der Position des Zehs in dem Tiefenbild, das in den sequentiell angezeigten Bildern enthalten ist.

13. Programm, das wenigstens einen Computer dazu veranlasst, das Messverfahren nach Anspruch 12 auszuführen.

**Revendications**

1. Dispositif de mesure (10) comprenant :

   une unité d'acquisition (11) adaptée pour acquérir des informations de position ou des informations de direction relatives à une partie prédéterminée d'un membre d'un sujet testé ;
   une unité de calcul (12) adaptée pour calculer des angles ou des distances servant d'indices de capacité du membre du sujet testé sur la base des informations de position ou de direction acquises ;

une unité de détermination (13) adaptée pour déterminer si une posture du sujet testé est correcte ou non, sur la base des informations de position ou de direction acquises ; et

une unité de traitement de sortie (14) adaptée pour délivrer en sortie les angles ou distances calculés dans un état correspondant à un résultat de détermination concernant le fait de savoir si la posture du sujet testé est correcte ou non ;

**caractérisé en ce que** l'unité d'acquisition (11) est adaptée pour acquérir des informations de position relatives au sol et des informations de position sur un orteil du sujet testé sur la base d'une image en profondeur d'une moitié inférieure du sujet testé qui est obtenue à partir d'un capteur tridimensionnel, et est adaptée pour acquérir des informations de position relatives à un bout du doigt de la main du sujet testé sur la base d'une image en profondeur du sujet testé, dans une région d'image ayant une coordonnée x plus grande que la coordonnée x de la position de l'orteil dans l'image en profondeur, dans laquelle l'orteil indique la direction positive de l'axe x dans un système de coordonnées de l'image en profondeur,

dans lequel l'unité de calcul (12) est adaptée pour calculer une distance entre le bout du doigt de la main et le sol sur la base des informations de position relatives au sol et des informations de position relative au bout du doigt de la main du sujet testé,

dans lequel le dispositif de mesure comprend en outre une unité d'acquisition d'images adaptée pour acquérir successivement des images comportant le sujet testé à partir du capteur tridimensionnel,

dans lequel l'unité de traitement de sortie (14) est adaptée pour superposer une marque sur la position de l'orteil du sujet testé sur les images acquises à afficher sur la base des informations de position acquises relatives l'orteil tout en affichant séquentiellement les images acquises, superposer de manière fixe la marque sur les images affichées séquentiellement par détection d'un événement prédéterminé après la superposition de la marque, et réaliser la coloration sur la base des informations de profondeur concernant le sujet testé sur une région d'image ayant une coordonnée x plus grande que la coordonnée x de la position de l'orteil dans l'image de profondeur incluse dans les images affichées séquentiellement.

2. Dispositif de mesure (10) selon la revendication 1, dans lequel l'unité de traitement de sortie (14) est en outre adaptée pour délivrer en sortie un affichage dans lequel un segment de ligne est superposé sur une image comportant le sujet testé dans un état correspondant au résultat de la détermination concernant le fait de savoir si la posture du sujet testé est correcte ou non, le segment de ligne reliant une pluralité de parties prédéterminées correspondant respectivement à une pluralité d'éléments d'informations de position utilisés pour le calcul des angles ou des distances.

3. Dispositif de mesure (10) selon la revendication 1 ou 2,
dans lequel l'unité d'acquisition (11) est adaptée pour acquérir séquentiellement les informations de position ou les informations de direction relatives à la partie prédéterminée dans le membre du sujet testé,
dans lequel l'unité de calcul (12) est adaptée pour calculer séquentiellement les angles ou les distances sur la base des informations de position ou des informations de direction acquises séquentiellement, et
dans lequel l'unité de traitement de sortie (14) est adaptée pour exclure un angle ou une distance calculé lorsqu'il est déterminé que la posture du sujet testé est incorrecte d'une sélection candidate d'une valeur maximale ou d'une valeur minimale parmi les angles ou les distances calculés séquentiellement avec par rapport au sujet testé lors de la délivrance en sortie de la valeur maximale ou de la valeur minimale.

4. Dispositif de mesure (10) selon l'une quelconque des revendications 1 à 3,
dans lequel l'unité d'acquisition (11) est adaptée pour acquérir des éléments d'informations de position relatives à une épaule et une main du sujet testé sur la base d'informations obtenues à partir d'un capteur tridimensionnel, et
dans lequel l'unité de calcul (12) est adaptée pour calculer en tant qu'indice de capacité d'un mouvement de flexion, d'un mouvement d'extension, d'un mouvement d'abduction ou d'un mouvement d'adduction de l'épaule sur la base des informations de position acquises, l'angle étant formé par un segment de ligne verticalement descendant d'un champ de vision du capteur tridimensionnel avec la position de l'épaule comme point final et un segment de ligne reliant une position de l'épaule et une position de la main, et l'angle étant sur un plan perpendiculaire à une direction horizontale ou une direction de profondeur du champ de vision du capteur tridimensionnel.

5. Dispositif de mesure (10) selon l'une quelconque des revendications 1 à 4,
dans lequel l'unité d'acquisition (11) est adaptée pour acquérir des éléments d'informations de position sur un coude et la main du sujet testé sur la base des informations obtenues à partir du capteur tridimensionnel, et
dans lequel l'unité de calcul (12) est adaptée pour calculer un angle en tant qu'indice de capacité d'un mouvement de rotation externe ou d'un mouvement de rotation interne de l'épaule sur la base des informations de position acquises, l'angle étant formé par un segment de ligne dans une direction opposée à la direction de profondeur du champ de vision du capteur tridimensionnel avec la position du coude comme point final et un segment de ligne reliant une position du coude et la position de la main, l'angle étant sur un plan perpendiculaire à une direction verticale du champ de vision du capteur tridimensionnel.

6. Dispositif de mesure (10) selon l'une quelconque des revendications 1 à 5,
dans lequel l'unité d'acquisition (11) est adaptée pour acquérir des éléments d'informations de position relatives à l'épaule et la main du sujet testé sur la base des informations obtenues à partir du capteur tridimensionnel, et
dans lequel l'unité de calcul (12) est adaptée pour calculer un angle en tant qu'indice de capacité d'un mouvement d'extension horizontale ou d'un mouvement de flexion horizontale de l'épaule sur la base des informations de position acquises, l'angle étant formé par un segment de ligne dans une direction horizontale du champ de vision du capteur tridimensionnel avec la position de l'épaule comme point final et un segment de ligne reliant la position de l'épaule et la position de la main, l'angle étant sur un plan perpendiculaire à la direction verticale du champ de vision du capteur tridimensionnel.

7. Dispositif de mesure (10) selon l'une quelconque des revendications 1 à 6,
dans lequel l'unité d'acquisition est adaptée pour acquérir des éléments d'informations de position relatives à une taille et un genou du sujet testé sur la base des informations obtenues à partir du capteur tridimensionnel, et
dans lequel l'unité de calcul (12) est adaptée pour calculer un angle en tant qu'indice de capacité d'un mouvement de flexion d'une hanche sur la base des informations de position acquises, l'angle étant formé par un segment de ligne verticalement descendant du champ de vision du capteur tridimensionnel avec la position de la taille comme point final et un segment de ligne reliant une position de la taille et une position du genou, l'angle étant sur un plan perpendiculaire à la direction horizontale du champ de vision du capteur tridimensionnel.

8. Dispositif de mesure (10) selon l'une quelconque des revendications 1 à 7,
dans lequel l'unité d'acquisition (11) est adaptée pour acquérir des informations de direction relatives à un visage et des informations de position relatives aux deux épaules du sujet testé sur la base des informations obtenues à partir du capteur tridimensionnel, et
dans lequel l'unité de calcul (12) est adaptée pour calculer un angle, qui est formé par un segment de ligne reliant les deux épaules et la direction horizontale du champ de vision du capteur tridimensionnel et est sur un plan perpendiculaire à la direction verticale du champ de vision du capteur tridimensionnel, en tant qu'angle d'ouverture de l'épaule sur la base des informations de position acquises, et pour calculer un angle servant d'indice de capacité d'un mouvement de rotation à droite ou d'un mouvement de rotation à gauche d'un cou sur la base des informations de direction acquises et de l'angle d'ouverture calculé de l'épaule.

9. Dispositif de mesure (10) selon la revendication 8, comprenant en outre :
une unité de rapport adaptée pour effectuer le rapport d'un résultat de détermination concernant le fait de savoir si l'angle d'ouverture calculé de l'épaule est inclus ou non dans une plage d'angles prédéterminée.

10. Dispositif de mesure (10) selon la revendication 8 ou 9, comprenant en outre :
dans lequel l'unité de détermination (13) est adaptée pour déterminer si l'angle d'ouverture calculé de l'épaule est inclus ou non dans une plage d'angles prédéterminée, et si le sujet testé est positionné ou non dans une plage de distances prédéterminée du capteur tridimensionnel.

11. Appareil de mesure (10) selon l'une quelconque des revendications 1 à 10,
dans lequel l'unité d'acquisition (11) est adaptée pour acquérir séquentiellement des images de profondeur d'un corps entier du sujet testé sur la base des informations obtenues à partir du capteur tridimensionnel, acquérir séquentiellement des éléments d'informations de position relatives au bout du doigt de la main du sujet testé sur la base des images de profondeur, et maintenir les informations de position relatives au bout du doigt de la main qui sont acquises pendant la détection de l'événement prédéterminé, et
dans lequel l'unité de calcul (12) est adaptée pour calculer une distance dans la direction horizontale du champ de vision du capteur tridimensionnel entre les bouts des doigts de la main, sur la base des informations de position maintenues relatives au bout du doigt de la main et des informations de position nouvellement acquises relatives au bout du doigt de la main.

12. Procédé de mesure exécuté par au moins un ordinateur, le procédé de mesure comprenant :

l'acquisition (S241) d'informations de position ou d'informations de direction relatives à une partie prédéterminée dans un membre d'un sujet testé ;

le calcul (S242) des angles ou des distances servant d'indices de capacité du membre du sujet testé sur la base des informations de position ou de direction acquises ;

la détermination (S243) si une posture du sujet testé est correcte ou non, sur la base des informations de position ou de direction acquises ; et

la délivrance en sortie (S244) des angles ou distances calculés dans un état correspondant à un résultat de détermination concernant le fait de savoir si la posture du sujet testé est correcte ou non ;

**caractérisé en ce que** l'acquisition (S241) d'informations de position ou d'informations de direction comprend l'acquisition d'informations de position relatives à un sol et d'informations de position relatives à un orteil du sujet testé sur la base d'une image en profondeur d'une moitié inférieure du sujet testé qui est obtenue à partir d'un capteur tridimensionnel, et l'acquisition d'informations de position relatives à un bout du doigt de la main du sujet testé sur la base d'une image en profondeur du sujet testé, dans une région d'image ayant une coordonnée x plus grande que la coordonnée x de la position de l'orteil dans l'image de profondeur, dans lequel l'orteil indique la direction positive de l'axe x d'un système de coordonnées de l'image de profondeur,

dans lequel le calcul (S242) d'angles ou de distances comprend le calcul d'une distance entre le bout du doigt de la main et le sol sur la base des informations de position relatives au sol et des informations de position relatives au bout du doigt de la main du sujet testé,

l'acquisition successive d'images comportant le sujet testé à partir du capteur tridimensionnel,

dans lequel la délivrance en sortie (S244) des angles ou distances calculés comprend la superposition d'une marque sur la position de l'orteil du sujet testé sur les images acquises à afficher sur la base des informations de position acquises relatives à l'orteil tout en affichant séquentiellement les images acquises, la superposition de manière fixe la marque sur les images affichées séquentiellement par détection d'un événement prédéterminé après la superposition de la marque, et l'exécution d'une coloration sur la base des informations de profondeur concernant le sujet testé sur une région d'image ayant une coordonnée x plus grande que la coordonnée x de la position de l'orteil dans l'image de profondeur incluse dans les images affichées séquentiellement.

**13.** Programme amenant au moins un ordinateur à exécuter le procédé de mesure selon la revendication 12.

EP 3 315 068 B1

FIG. 1

10

MEASUREMENT DEVICE

5
DISPLAY
DEVICE

1
CPU

3
INPUT AND
OUTPUT I/F

INPUT DEVICE

6

7

2
MEMORY

4
COMMUNICATION
UNIT

THREE-
DIMENSIONAL
SENSOR

34

FIG. 2

MEASUREMENT DEVICE 10

CALCULATION UNIT 12

OPERATION DETECTION UNIT 15

DATA ACQUISITION UNIT 11

OUTPUT PROCESSING UNIT 14

DETERMINATION UNIT 13

# FIG. 3

| SHOULDER | FLEXION MOTION / EXTENSION MOTION | |
| | ABDUCTION MOTION / ADDUCTION MOTION | |
| | EXTERNAL ROTATION MOTION / INTERNAL ROTATION MOTION | |
| | HORIZONTAL EXTENSION MOTION / HORIZONTAL FLEXION MOTION | |

FIG. 4

D2

MOVABLE RANGE

D21

PORTION NAME : SHOULDER

MOTION DIRECTION : FLEXION (FORWARD RAISE-UP)

REFERENCE MOVABLE RANGE ANGLE :180°

D22
150°

D23 MAXIMUM VALUE :150°

D24 MINIMUM VALUE : —

FLEXION

0°

D1

CAMERA IMAGE

Start

EP 3 315 068 B1

37

FIG. 5

MOVABLE RANGE

PORTION NAME : SHOULDER

MOTION DIRECTION : ABDUCTION (SIDEWARD RAISE-UP)

REFERENCE MOVABLE RANGE ANGLE :180°

125°

MAXIMUM VALUE :141°

MINIMUM VALUE : —

ABDUCTION

0°

CAMERA IMAGE

Start

# FIG. 6

**D2** — MOVABLE RANGE

**D21**
- PORTION NAME : SHOULDER
- MOTION DIRECTION : EXTERNAL ROTATION
- REFERENCE MOVABLE RANGE ANGLE : 60°

**D22** — 51°

**D23** — MAXIMUM VALUE : 67°

**D24** — MINIMUM VALUE : —

EXTERNAL ROTATION 0°

**D1** — CAMERA IMAGE

Start

EP 3 315 068 B1

FIG. 7

MOVABLE RANGE — □ ✕

D21 — PORTION
NAME : SHOULDER

MOTION : HORIZONTAL
DIRECTION FLEXION

REFERENCE MOVABLE
RANGE ANGLE :135°

D22 — **145°**

D23 — MAXIMUM : **150°**
VALUE

D24 — MINIMUM : —
VALUE

0°

HORIZONTAL FLEXION

CAMERA IMAGE — □ ✕

Start

D2

D1

FIG. 8

START

ACQUIRE FRAMEWORK DATA — S81

CALCULATE ANGLE — S82

DETERMINE WHETHER
OR NOT POSTURE OF
TEST SUBJECT IS PROPER — S83

OUTPUT ANGLE — S84

IS POSTURE PROPER AND
IS ANGLE MAXIMUM VALUE? — S85
NO

YES

OUTPUT MAXIMUM VALUE — S86

OUTPUT MARK OF
PREDETERMINED PORTION — S87

OUTPUT LINE SEGMENT — S88

END

FIG. 9

| THORACIC AND LUMBAR SPINE | FLEXION MOTION | |
|---|---|---|

# FIG. 10

D2 — MOVABLE RANGE

D21 —
PORTION NAME : THORACIC AND LUMBAR SPINES

MOTION DIRECTION : FLEXION

D22 — cm
D23 — MAXIMUM VALUE : —
D24 — MINIMUM VALUE : —

D1 — CAMERA IMAGE

Start

MK1

EP 3 315 068 B1

## FIG. 11

EP 3 315 068 B1

FIG. 12

START

ACQUIRE FRAMEWORK DATA — S121

ACQUIRE DEPTH IMAGE — S122

OUTPUT DEPTH IMAGE OF LOWER HALF OF TEST SUBJECT — S123

ACQUIRE POSITIONAL INFORMATION ON FLOOR — S124

ACQUIRE POSITIONAL INFORMATION ON TOE — S125

OUTPUT MARK OF PREDETERMINED PORTION — S126

OUTPUT LINE SEGMENT — S127

OUTPUT MARK OF TOE (FLOOR) — S128

IS PREDETERMINED EVENT DETECTED? — S129

NO

YES

FIX MARK OF TOE (FLOOR) — S130

TO FIG. 13

# FIG. 13

START

S131
ACQUIRE FRAMEWORK DATA

S132
ACQUIRE DEPTH IMAGE

S133
OUTPUT DEPTH IMAGE ON OUTSIDE OF TOE OF TEST SUBJECT

S134
ACQUIRE POSITIONAL INFORMATION ON FINGERTIP OF HAND

S135
OUTPUT DISTANCE BETWEEN FINGERTIP OF HAND AND FLOOR

S136
DETERMINE WHETHER OR NOT POSTURE IS PROPER

S137
OUTPUT DISTANCE

S138
IS POSTURE PROPER AND IS DISTANCE MINIMUM VALUE?

NO

YES

S139
OUTPUT MINIMUM VALUE

S140
OUTPUT MARK OF PREDETERMINED PORTION

S141
OUTPUT LINE SEGMENT

END

# FIG. 14

| | |
|---|---|
| **MOVABLE RANGE** | **CAMERA IMAGE** |
| FUNCTIONAL REACH TEST | Start |
| **33** cm | |
| MAXIMUM VALUE : 38cm | |
| MINIMUM VALUE : — | |

D2 · D1 · D21 · D22 · D23 · D24

EP 3 315 068 B1

EP 3 315 068 B1

FIG. 15

START

ACQUIRE DEPTH IMAGE — S151

OUTPUT DEPTH IMAGE OF WHOLE
BODY OF TEST SUBJECT — S152

ACQUIRE POSITIONAL
INFORMATION ON FINGERTIP OF
HAND — S153

OUTPUT MARK OF
FINGERTIP OF HAND — S154

IS PREDETERMINED
EVENT DETECTED? — S155

NO

YES

HOLD POSITIONAL INFORMATION
ON FINGERTIP OF HAND — S156

FIX MARK OF FINGERTIP OF HAND — S157

FIX DEPTH IMAGE OF WHOLE BODY — S158

TO FIG. 16

FIG. 16

START

ACQUIRE DEPTH IMAGE — S161

OUTPUT DEPTH IMAGE OF
WHOLE BODY OF TEST SUBJECT — S162

ACQUIRE POSITIONAL
INFORMATION ON FINGERTIP
OF HAND — S163

CALCULATE DISTANCE
BETWEEN FINGERTIPS OF
HAND — S164

DETERMINE WHETHER OR
NOT POSTURE IS PROPER — S165

OUTPUT DISTANCE — S166

IS POSTURE PROPER
AND IS DISTANCE MAXIMUM
VALUE? — S167

NO

YES

OUTPUT MAXIMUM VALUE — S168

OUTPUT MARK OF
FINGERTIP OF HAND — S169

END

FIG. 17

MEASUREMENT DEVICE

FIG. 18

# FIG. 19

D2 — MOVABLE RANGE

D21 —
PORTION NAME : CERVICAL SPINES
MOTION DIRECTION : RIGHT ROTATION
REFERENCE MOVABLE RANGE ANGLE :60°

D22 — **56°**

D23 — MAXIMUM VALUE :59°
D24 — MINIMUM VALUE : —

0° RIGHT ROTATION x

D1 — CAMERA IMAGE

Start

D15   D16

[1.65]m [32.7]

Please sit 1.5 to 2.0 meters away

EP 3 315 068 B1

EP 3 315 068 B1

## FIG. 20

START

ACQUIRE FRAMEWORK DATA — S201

OUTPUT LINE SEGMENT OF BOTH SHOULDERS — S202

CALCULATE OPENING ANGLE BETWEEN BOTH SHOULDERS — S203

OUTPUT OPENING ANGLE BETWEEN BOTH SHOULDERS — S204

ACQUIRE DEPTH IMAGE OF TEST SUBJECT — S205

OUTPUT DEPTH OF TEST SUBJECT — S206

IS OPENING ANGLE BETWEEN BOTH SHOULDERS PROPER? — S207
NO / YES

REPORTING — S208

ACQUIRE DIRECTION INFORMATION ON FACE — S209

CALCULATE ANGLE — S210

OUTPUT ANGLE — S211

IS POSTURE PROPER AND IS ANGLE MAXIMUM VALUE? — S212
NO / YES

OUTPUT MAXIMUM VALUE — S213

END

53

FIG. 21

FIG. 22

EP 3 315 068 B1

FIG. 23

FIG. 24

```
        ┌─────────┐
        │  START  │
        └────┬────┘
             ↓
┌───────────────────────────┐
│ ACQUIRE POSITIONAL INFORMATION │  ⌇ S241
│    OR DIRECTION INFORMATION    │
└───────────────┬───────────────┘
                ↓
┌───────────────────────────┐
│ CALCULATE ANGLE OR DISTANCE │      ⌇ S242
└───────────────┬───────────────┘
                ↓
┌───────────────────────────┐
│      DETERMINE WHETHER       │       ⌇ S243
│    OR NOT POSTURE IS PROPER   │
└───────────────┬───────────────┘
                ↓
┌───────────────────────────────────────────┐
│      OUTPUT ANGLE OR DISTANCE IN STATE       │  ⌇ S244
│  CORRESPONDING TO DETERMINATION RESULT       │
│ REGARDING WHETHER OR NOT POSTURE IS PROPER   │
└───────────────────┬───────────────────────┘
                    ↓
              ┌─────────┐
              │   END   │
              └─────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015061579 A

**Non-patent literature cited in the description**

- Joint Movable Range Display and Measurement Method. *Japanese Orthopaedic Assoc. and Japanese Assoc. of Rehabilitation Medicine, Rehabilitation Medicine,* 1995, vol. 32, 207-217

- **KITSUNEZAKI NAOFUMI.** KINECT Applications for the Physical Rehabilitation. *the Institute of Electronics, Information and Communication Engineers, IEICE technical report, IE2012-89,* November 2012
- Physio@Home. **RICHARD TANG et al.** HUMAN FACTORS IN COMPUTING SYSTEMS. ACM, 18 April 2015